# EUROPEAN PATENT APPLICATION

(11) **EP 4 353 832 A1**
(43) Date of publication of application: **17.04.2024**
(21) Application number: 21944432.0
(22) Date of filing: 12.11.2021
(51) Int. Cl.: C12Q 1/6809, G16B 20/50

(54) **METHOD AND KIT FOR DETECTING N6-METHYLADENOSINE IN NUCLEIC ACID MOLECULES**

(30) Priority: 11.06.2021 CN 202110655085
(71) Applicant: Peking University, Beijing 100871 (CN)
(72) Inventor: YI, Chengqi, Beijing 100871 (CN); WANG, Jing, Beijing 100871 (CN)
(74) Representative: E. Blum & Co. AG
(86) International application number: PCT/CN2021/130282
(87) International publication number: WO 2022/257354

(57) **Abstract**

The present application provides a method and kit for detecting N6-methyladenine in a nucleic acid molecule.

## Description

The present application is based on the CN application with the application number 202110655085.7 and the filing date of June 11, 2021, and claims its priority. The disclosure content of the CN application is hereby incorporated into the present application in its entirety.

### Technical Field

The present application relates to the field of molecular biology, especially the field of nucleic acid detection and analysis. In particular, the present application provides a method for detecting N6-methyladenine in nucleic acid molecules. In addition, the present application also provides a kit, which can be used to implement the method of the present application.

### Background Art

The development of high-throughput sequencing technology is of great significance for studying the biological functions of epigenetic modifications. Currently, a variety of sequencing methods have been developed for N6-methyladenine (m⁶A) modification. Among them, MeRIP-seq (i.e., m⁶A-seq) is currently the most widely used sequencing method for m⁶A at the whole transcriptome level. This method uses m⁶A-specific antibodies to enrich RNA fragments containing m⁶A, and then performs sequencing analysis, and its resolution is around 200nt. miCLIP, based on the combination of antibody cross-linking and immunoprecipitation, enables accurate localization of m⁶A residues at single-base resolution through mutation or truncation information. The subsequently developed m⁶ACE-seq, by using antibody cross-linking and exonuclease digestion, can also achieve m⁶A sequencing at single-base resolution. The above methods all rely on m⁶A-specific antibodies, have poor reproducibility, require large amounts of samples, and are difficult to quantitatively detect m⁶A modifications. Two methods, m⁶A-REF-seq and MAZTER-seq, use RNA endonucleases and do not rely on antibodies to achieve quantitative m⁶A sequencing. However, these two methods can only detect specific m⁶A modifications and cannot analyze all m⁶A modifications.

Recently, disclosed in Chem. Sci., 2021, 12, 606 is a method of using an acetic acid aqueous solution of sodium nitrite to achieve the conversion of adenine (A) to hypoxanthine (I) in nucleic acids (DNA/RNA), in which during the amplification or reverse transcription process, I is recognized as guanine (G), while methyladenine is still read as A, and it can be determined whether the nucleic acid contains m⁶A modification by comparing the conversion of A to G. However, the overall reaction efficiency of this method does not exceed 20%, so it cannot quantitatively analyze low-abundance m⁶A sites, and there is a large amount of conversion of cytosine (C) to uracil (U) in this method. Moreover, the use of acetic acid aqueous solution in this method can also lead to the degradation of nucleic acid.

NOseq (Nucleic Acids Res. 2021 Feb 26;49(4):e23) also uses nitroso compounds to treat A to deaminate it into I and be read as G, while m⁶A is converted to NO-m⁶A and cannot be deaminated and is still recognized as A, thereby distinguishing the m⁶A site. The conversion efficiency of A-to-I in this method is between 10% and 50%, and a large amount of A residues remains at the conventional A sites after nitroso compound treatment. Therefore, there is a large amount of background noise in the analysis of m⁶A, and low-abundance methylation sites cannot be analyzed. At the same time, this method can only identify certain methylation sites in specific sequences and cannot detect m⁶A modifications in the whole transcriptome. Moreover, this method still has a large number of false positive or non-specific conversions, for example, G will be deaminated and converted into xanthine (X), while X will lead to transcription termination, or lead to false positives when X is mismatched and be read as A, and C will also be deaminated and converted into U, which brings difficulties for subsequent analysis.

In summary, currently existing m⁶A sequencing methods are unable to achieve a quantitative analysis of m⁶A at the whole transcriptome level. Therefore, there is an urgent need to develop a quantifiable and highly sensitive detection method for N6-methyladenine at the whole genome or whole transcriptome level.

### Contents of the Invention

Based on in-depth research, the present inventors of the present application developed a new method for detecting N6-methyladenine in nucleic acid molecules. The method of the present application utilizes a nitrite and a carbonyl compound to catalyze the deamination of adenine into hypoxanthine, while N6-methyladenine cannot be deaminated due to its stable chemical properties. Therefore, the method of the present application realizes the detection of the m⁶A modification level or m⁶A site by analyzing the signals of adenine or hypoxanthine before and after conversion. This method is highly sensitive, quantitatively accurate, and can detect N6-methyladenine in the whole genome or transcriptome at single-base resolution.

Therefore, in one aspect, the present application provides a method for detecting N6-methyladenine in a nucleic acid molecule, which comprises the following steps:
(1) performing protection of an amino group of guanine in a nucleic acid molecule to be detected to obtain a protected nucleic acid molecule;
(2) reacting the protected nucleic acid molecule with a nitrite in the presence of a carbonyl compound to convert an adenine in the nucleic acid molecule to be detected into a hypoxanthine;
(3) optionally, performing deprotection of the product obtained in step (2);
(4) detecting the product obtained in the previous step.

It is easy to understand that in step (1) of the method, any base other than adenine in the nucleic acid molecule can be protected by any method, wherein the protection can prevent or hinder the deamination reaction of the protected base. In certain embodiments, the protection is reversible or irreversible.

In certain embodiments, the protection is irreversible, and the protected base is capable of performing complementary base pairing.

In the method of the present application, a nitrite is used to deaminate the adenine in the nucleic acid molecule to be detected and convert it into hypoxanthine. However, N6-methyladenine cannot be deaminated due to its stable chemical properties. Therefore, the m⁶A modification level or m⁶A site can be detected by analyzing the signals of adenine or hypoxanthine before and after the conversion.

Therefore, it can be understood that in certain embodiments, the method can detect the m⁶A modification level by directly detecting the signals of adenine or hypoxanthine in the product of the nucleic acid to be detected undergoing the catalytic deamination reaction. In certain embodiments, the detection of the m⁶A modification level can be achieved by detecting the signals of adenine or hypoxanthine in the product of the nucleic acid to be detected undergoing the catalytic deamination reaction by methods such as mass spectrometry, enzyme fragmentation, and/or chromatography.

Because hypoxanthine is complementary to cytosine during reverse transcription or DNA replication, it is read as guanine; N6-methyladenine remains complementary to thymine or uracil, and is still read as adenine. Therefore, in certain embodiments, the method can detect the m⁶A modification level and m⁶A site by detecting the sequence information of the amplification product or the reverse transcription product of the product of the nucleic acid to be detected undergoing the catalytic deamination reaction. In some embodiments, the method can detect the m⁶A modification level and m⁶A site by detecting the sequence information of the amplification product or the reverse transcription product of the product of the nucleic acid to be detected undergoing the catalytic deamination reaction by methods such as sequencing or hybridization.

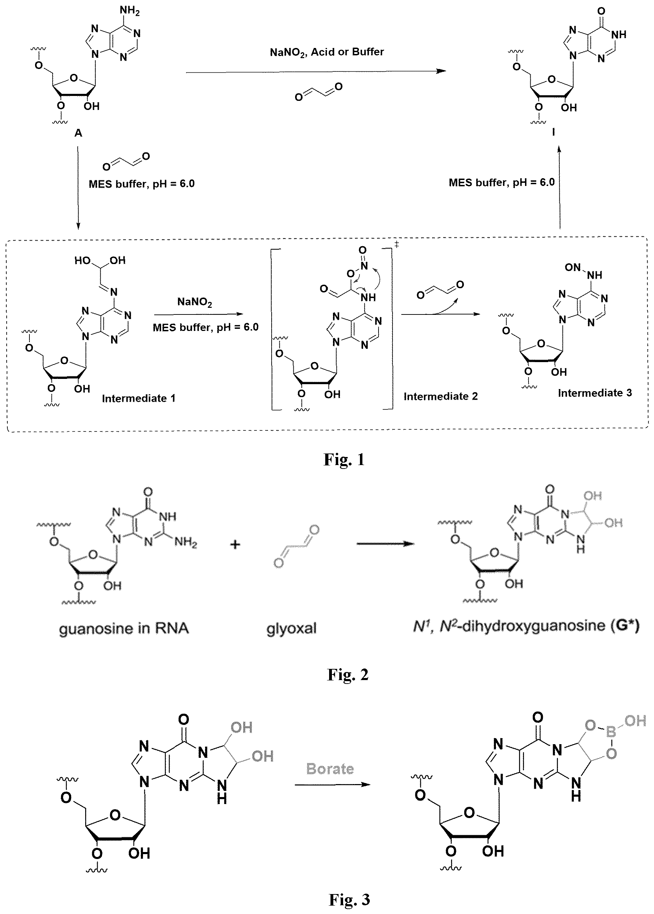

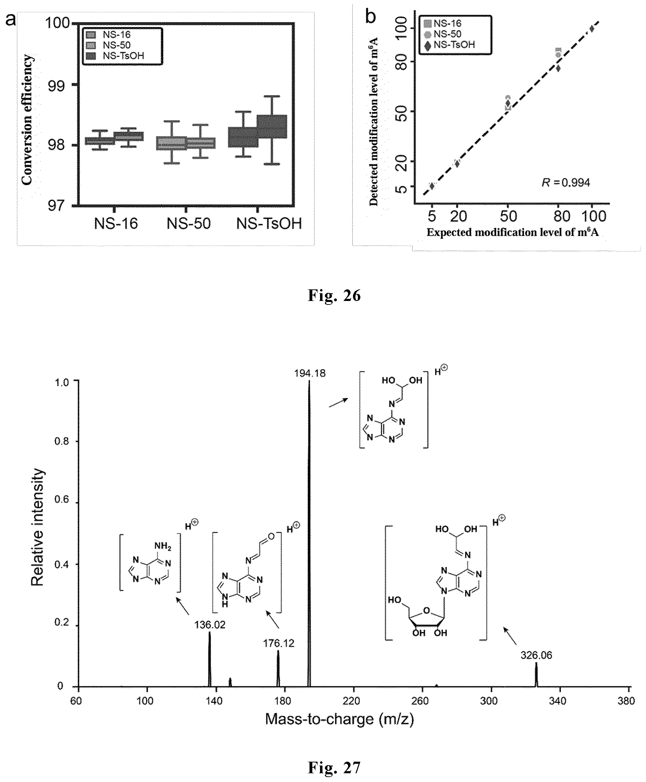

Without being limited by any theory, the present inventors speculate that in the method of the present invention, the nitrite is used to deaminate adenine in the nucleic acid molecule to be detected and convert it into hypoxanthine. In the process of converting adenine (A) to hypoxanthine (I), a carbonyl compound (e.g., glyoxal) plays a catalytic role and can effectively improve the conversion efficiency of A-to-I in the nucleic acid molecule. Taking glyoxal as an example, the exemplary catalytic reaction mechanism is shown in Fig. 1. During the reaction process, the present inventors detected an imine intermediate formed by reacting glyoxal with the amino of adenine, and its secondary mass spectrum is shown in Fig. 27.

Guanine will be converted into xanthine (X) during the nitrosation reaction, and xanthine will cause the termination of the nucleic acid amplification or reverse transcription process at a certain probability, or xanthine will perform complementary pairing with thymine during the nucleic acid amplification or reverse transcription process, and thus be read as adenine, interfering with the detection of the m⁶A modification level and m⁶A site. Therefore, in certain embodiments, it is particularly advantageous to protect the amino group of guanine in the nucleic acid molecule to be detected.

In certain embodiments, in step (1) of the method, a second carbonyl compound is used to protect the amino group of guanine in the nucleic acid molecule to be detected.

When a second carbonyl compound (taking glyoxal as an example) is used to protect the amino group of guanine in the nucleic acid molecule to be detected, the reaction mechanism is shown in Fig. 2. The second carbonyl compound, such as glyoxal, can react with the N¹ and N² positions of guanine to form N¹,N²-dihydroxyguanosine, thereby reducing the deamination efficiency of guanine.

In certain embodiments, the carbonyl compound is from:
(i) a second carbonyl compound added in step (1);
(ii) a first carbonyl compound added in step (2); or,
(iii) a combination of (i) and (ii).

In certain embodiments, the second carbonyl compound is the same as or different from the first carbonyl compound. In certain embodiments, the second carbonyl compound is the same as the first carbonyl compound.

In certain embodiments, step (2) does not comprise a step of adding a first carbonyl compound, and the carbonyl compound is from a second carbonyl compound added in step (1); or, step (2) comprises a step of adding a first carbonyl compound, and the first carbonyl compound is the same as or different from the second carbonyl compound.

In certain embodiments, in step (3), guanine in the product is optionally undergoes deprotection.

In certain embodiments, the first carbonyl compound and the second carbonyl compound are each independently selected from the group consisting of compounds represented by Formula I and any combination thereof, 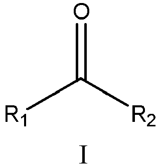
wherein R₁ is an aldehyde group, C₁₋₃ alkyl (e.g., methyl, ethyl or n-propyl), or halogen-substituted C₁₋₃ alkyl (e.g., trichloromethyl, trifluoromethyl), R₂ is H, C₁₋₃ alkyl (e.g., methyl, ethyl or n-propyl), phenyl or HC(=O)-CH(Br)-; or,
R₁ and R₂ together with the carbonyl group to which they are bonded form a cyclic structure, such as ninhydrin.

In certain embodiments, the first carbonyl compound and the second carbonyl compound are each independently selected from: glyoxal, 2,3-butanedione, ninhydrin, 2-bromomalondialdehyde, pyruvaldehyde, trichloroacetaldehyde, phenylglyoxal and any combination thereof.

In certain embodiments, the first carbonyl compound is selected from the group consisting of: glyoxal, 2,3-butanedione, ninhydrin, 2-bromomalondialdehyde, pyruvaldehyde, trichloroacetaldehyde, phenylglyoxal and any combination thereof.

In certain embodiments, the first carbonyl compound is selected from the group consisting of glyoxal, 2-bromomalondialdehyde, 2,3-butanedione, phenylglyoxal, trichloroacetaldehyde, ninhydrin, and any combination thereof.

In certain embodiments, the second carbonyl compound is selected from the group consisting of: glyoxal, ninhydrin, and any combination thereof.

In certain embodiments, in step (1) of the method, the nucleic acid molecule to be detected is contacted with the second carbonyl compound in a first solvent.

In certain embodiments, the first solvent is water.

In certain embodiments, in step (1), the nucleic acid molecule to be detected is contacted with the second carbonyl compound in the first solvent, and in the presence of dimethylsulfoxide (DMSO) or N,N-dimethylformamide (DMF).

In certain embodiments, in step (1), the nucleic acid molecule to be detected is contacted with the second carbonyl compound in the first solvent, and in the presence of dimethylsulfoxide (DMSO) and boric acid or a salt thereof (e.g., potassium borate).

In certain embodiments, in step (1), the nucleic acid molecule to be detected is contacted with the second carbonyl compound in the first solvent, and in the presence of N,N-dimethylformamide (DMF) and boric acid or a salt thereof (e.g., potassium borate).

In some embodiments, in step (1), the dimethylsulfoxide (DMSO) has a final concentration of 20 v/v% to 90 v/v%, such as about 30 v/v%, about 40 v/v%, about 50 v/v%, about 60 v/v%, about 70 v/v%, about 80 v/v%.

In some embodiments, in step (1), the boric acid or salt thereof (e.g., potassium borate) has a final concentration of 5mM to 150 mM (e.g., 5mM to 10 mM, 10 mM to 50 mM, 50 mM to 80 mM, 80 mM to 100 mM).

In some embodiments, in step (1), the second carbonyl compound has a final concentration of greater than or equal to 20 mM, preferably 20 mM to 3M (e.g., 20 mM to 100 mM, 100 mM to 500 mM, 500 mM to 1000 mM, 1M to 1.5M, 1.5 to 2M, 2M to 2.6M, for example, about 0.8M, about 1.3M, about 1.8M).

In certain embodiments, in step (1) of the method, the nucleic acid molecule to be detected is contacted with the second carbonyl compound under a temperature of 16°C to 60°C (e.g., about 20°C, about 25°C, about 30°C, about 35°C, about 40°C, about 45°C, about 50°C, about 55°C).

In certain embodiments, in step (1) of the method, the nucleic acid molecule to be detected is contacted with the second carbonyl compound under a temperature of 16°C to 60°C (e.g., about 20°C, about 25°C, about 30°C, about 35°C, about 40°C, about 45°C, about 50°C, about 55°C) for 15 to 60 minutes (e.g., about 20minutes, about 30minutes, about 40minutes, about 50min).

In some embodiments, the method further comprises: before step (1), a step of performing pretreatment of the nucleic acid molecule to be detected.

In certain embodiments, the pretreatment comprises purification, fragmentation, denaturation, or any combination thereof of the nucleic acid molecule to be detected.

In certain embodiments, the pretreatment comprises denaturation of the nucleic acid molecule to be detected using an aqueous formamide solution or an alkali solution.

In certain embodiments, in step (2) of the method, in the presence of the carbonyl compound, the protected nucleic acid molecule is reacted with the nitrite in a second solvent.

In certain embodiments, the second solvent is selected from the group consisting of water, 2-(N-morpholino)ethanesulfonic acid (MES) buffer solution, sodium acetate buffer solution, 3-(N-morpholino)propanesulfonic acid (MOPS) buffer solution, piperazine-1,4-diethanesulfonic acid (PIPES) buffer solution, 4-hydroxyethylpiperazineethanesulfonic acid (HEPPS) buffer solution or tris(hydroxymethyl)aminomethane (TRIS) buffer solution, and any combination thereof. The second solvent may also be a p-toluenesulfonic acid aqueous solution or a phosphoric acid aqueous solution.

In certain embodiments, the second solvent is water, p-toluenesulfonic acid aqueous solution, phosphoric acid aqueous solution, 2-(N-morpholino)ethanesulfonic acid (MES) buffer solution, sodium acetate buffer solution, 3-(N-morpholino)propanesulfonic acid (MOPS) buffer solution, piperazine-1,4-diethanesulfonic acid (PIPES) buffer solution, 4-hydroxyethylpiperazineethanesulfonic acid (HEPPS) buffer solution or tris(hydroxymethyl)aminomethane (TRIS) buffer solution, or any combination thereof.

In certain embodiments, the second solvent is 2-(N-morpholino)ethanesulfonic acid (MES) buffer solution or sodium acetate buffer solution.

In certain embodiments, the second solvent is p-toluenesulfonic acid aqueous solution, 2-(N-morpholino)ethanesulfonic acid (MES) buffer solution, or sodium acetate buffer solution.

In certain embodiments, the second solvent is p-toluenesulfonic acid aqueous solution, phosphoric acid aqueous solution, 2-(N-morpholino)ethanesulfonic acid (MES) buffer solution, or sodium acetate buffer solution.

In certain embodiments, the second solvent is a p-toluenesulfonic acid aqueous solution at a pH of 4.5 to 6.5 (e.g., 5 to 6).

In certain embodiments, the second solvent is a phosphoric acid aqueous solution at a pH of 4.5 to 6.5 (e.g., 5 to 6).

In certain embodiments, the second solvent is 2-(N-morpholino)ethanesulfonic acid (MES) buffer solution, wherein the 2-(N-morpholino)ethanesulfonic acid (MES) has a final concentration of 20 mM to 750 mM (e.g., about 40 mM, about 80 mM, about 100 mM, about 150 mM, about 200 mM, about 250 mM, about 500 mM).

In certain embodiments, the second solvent is a sodium acetate buffer solution, wherein the sodium acetate has a final concentration of 300 mM to 400 mM.

In certain embodiments, in step (2), in the presence of the carbonyl compound, the protected nucleic acid molecule is reacted with the nitrite in the second solvent, and in the presence of boric acid or a salt thereof (e.g., potassium borate).

In certain embodiments, the carbonyl compound in the second solvent has a total molar concentration (final concentration) of greater than or equal to 50 mM, such as greater than or equal to 250 mM, (e.g., 50 to 2000 mM, 50 to 200 mM, 200 to 500 mM, 500 to 800 mM, 800 to 1200 mM, 1200 to 1400 mM, 1400 to 1800 mM, 1800 to 2000 mM, e.g., 105 mM).

In certain embodiments, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, e.g., 105 mM) and pyruvaldehyde at a final concentrations of 50 to 1500 mM (e.g., 50 to 60 mM, 60 to 100 mM, 100 to 300 mM, 300 to 600 mM, 600 to 800 mM, 800 to 1200 mM, 1200 to 1500 mM).

In certain embodiments, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, e.g., 105 mM) and 2,3-butanedione at a final concentrations of 50 to 2500 mM (e.g., 50 to 100 mM, 100 to 200 mM, 200 to 500 mM, 500 to 800 mM, 800 to 1200 mM, 1200 to 1500 mM, 1500 to 1800 mM, 1800 to 2200 mM, 2200 to 2500 mM).

In certain embodiments, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, e.g., 105 mM) and ninhydrin at a final concentration of 20 to 1500 mM (e.g., 20 to 100 mM, 100 to 200 mM, 200 to 500 mM, 500 to 800 mM, 800 to 1200 mM, 1200 to 1500 mM, Approximately 60 mM).

In certain embodiments, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, e.g., 105 mM) and 2-bromomalondialdehyde at a final concentration of 50 to 1500 mM (e.g., 50 to 100 mM, 100 to 200 mM, 200 to 400 mM, 400 to 500 mM, 500 to 800 mM, 800 to 1200 mM, 1200 to 1500 mM).

In certain embodiments, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, e.g., 105 mM) and trichloroacetaldehyde at a final concentration of 50 to 1500 mM (e.g., 50 to 100 mM, 100 to 400 mM, 400 to 500 mM, 500 to 800 mM, 800 to 1200 mM, 1200 to 1500 mM).

In certain embodiments, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, e.g., 105 mM) and phenylglyoxal at a final concentration of 50 to 1500 mM (e.g., 50 to 100 mM, 100 to 400 mM, 400 to 500 mM, 500 to 800 mM, 800 to 1200 mM, 1200 to 1500 mM).

In certain embodiments, the second solvent contains glyoxal at a final concentration of greater than or equal to 50 mM, such as greater than or equal to 250 mM (e.g., 50 to 2000 mM, 50 to 200 mM, 200 to 500 mM, 500 to 800 mM, 800 to 1200 mM, 1200 to 1400 mM, 1400 to 1800 mM, 1800 to 2000 mM, e.g., 105mM).

In some embodiments, in step (2), the boric acid or salt thereof (e.g., potassium borate) has a final concentration of 5 mM to 150 mM (e.g., 5 mM to 10 mM, 10 mM to 50 mM, 50 mM to 80 mM, 80 mM to 100 mM).

In certain embodiments, the nitrite is selected from the group consisting of sodium nitrite, potassium nitrite, and combinations thereof.

In certain embodiments, the nitrite has a final concentration of 0.5 M to 1.5 M, such as 0.5 M to 0.8 M, 0.8 to 1.25 M.

In certain embodiments, in step (2) of the method, in the presence of the carbonyl compound, the protected nucleic acid molecule is reacted with the nitrite in a second solvent, and in the presence of boric acid or salt thereof (e.g., potassium borate).

The N¹,N²-dihydroxyguanosine formed when the amino group of guanine in the nucleic acid molecule is protected by a second carbonyl compound such as glyoxal may have its protecting group removed in step (2). As shown in Fig. 3, adding boric acid or salt thereof (e.g., H₃BO₃/K₃BO₃) in step (2) can further protect N¹,N²-dihydroxyguanosine, making its structure more stable and making the protective group difficult to remove.

In certain embodiments, the boric acid or salt thereof (e.g., potassium borate) is from:
(i) a boric acid or salt thereof (e.g., potassium borate) added in step (1);
(ii) a boric acid or salt thereof (e.g., potassium borate) added in step (2);
(iii) a boric acid or salt thereof (e.g., potassium borate) added after step (1) and before step (2); or,
(iv) any combination of (i) to (iii).

In certain embodiments, step (2) does not comprise a step of adding boric acid or salt thereof (e.g., potassium borate); or, step (2) comprises a step of adding boric acid or salt thereof (e.g., potassium borate), and, the boric acid or salt thereof (e.g., potassium borate) added in step (2) is the same as or different from the boric acid or salt thereof (e.g., potassium borate) added in step (1).

It is easy to understand that the way of adding boric acid or salt thereof in the second solvent is not limited by the reaction step. For example, the boric acid or salt thereof can be added before step (1) (e.g., incubating boric acid or salt thereof with the second carbonyl compound, and then performing the nucleic acid protection process of step (1)); for example, the boric acid or salt thereof can be directly added in step (1); for example, the boric acid or salt thereof can be directly added in step (2); for example, the boric acid or salt thereof can be added to the protected nucleic acid after step (1) and before step (2).

In certain embodiments, in step (2) of the method, the protected nucleic acid molecule is reacted with the nitrite at a temperature of 12 to 60°C (e.g., 16 to 60°C, 12 to 24°C, 24 to 40°C, 40 to 60°C, about 16°C, about 20°C, about 25°C, about 30°C, about 35°C, about 40°C, about 45°C, about 50°C, about 55 °C).

In some embodiments, in step (2), the protected nucleic acid molecule is reacted with the nitrite at a temperature of 12 to 60°C (e.g., 16 to 60°C, 12 to 24°C, 24 to 40°C, 40 to 60°C, about 16°C, about 20°C, about 25°C, about 30°C, about 35°C, about 40°C, about 45°C, about 50°C, about 55°C) for 10 minutes to 24 hours (e.g., 30 minutes to 24 hours, 10 minutes to 20 minutes, 20 minutes to 1h, 1h to 5h, 5h to 10h, 10h to 24h, about 20 minutes, about 30 minutes, about 40 minutes, about 50 minutes, about 8 hours).

In some embodiments, in step (2), the protected nucleic acid molecule is reacted with the nitrite at a temperature of 12-24°C (e.g., about 16°C) for 5h to 10h (e.g., about 8h).

In some embodiments, in step (2), the protected nucleic acid molecule is reacted with the nitrite at a temperature of 40 to 60°C (e.g., about 50°C) for 20 minutes to 1 hour (e.g., about 30 min).

In some embodiments, the method further comprises: before step (4), a step of performing purification, reverse transcription, and/or amplification of the product obtained in the previous step.

In certain embodiments, the method further comprises an additional step of detecting the nucleic acid molecule to be detected.

In certain embodiments, in step (4), the detection comprises performing nucleotide composition analysis by sequencing or hybridization, mass spectrometry (e.g., triple tandem quadrupole mass spectrometry), enzymatic fragmentation, and/or chromatography.

In certain embodiments, the detection comprises performing nucleotide composition analysis by sequencing.

In some embodiments, the method further comprises comparing the detection results of step (4) with the detection results of the additional step (e.g., comparing the sequence or nucleotide composition of the product obtained in step (4) with the sequence or nucleotide composition of the nucleic acid molecule to be detected obtained in the additional step, and determining the content and/or position information of N6-methyladenine in the nucleic acid molecule to be detected).

In certain embodiments, the nucleic acid molecule to be detected can be derived from any organism (e.g., eukaryotic cell, prokaryotic cell, virus, and viroid) or non-organism (e.g., nucleic acid molecule library). The nucleic acid molecule to be detected may exist in single-stranded or double-stranded form.

In certain embodiments, in the method, the nucleic acid molecule to be detected is an RNA, a DNA, or a DNA/RNA hybrid.

In some embodiments, in step (3), the product obtained in step (2) undergoes deprotection under an alkaline condition; or, the product obtained in step (2) undergoes deprotection in a phosphate buffer solution.

In certain embodiments, in step (3), the product obtained in step (2) undergoes deprotection by a heat treatment under an alkaline condition.

In certain embodiments, the product obtained in step (2) undergoes deprotection by a heat treatment in a triethylamine acetate (TEAA) buffer solution containing formamide at a pH of 8-9 or in a phosphate buffer solution containing dimethylsulfoxide (DMSO) at a pH of 7.1-8.

In certain embodiments, the product obtained in step (2) undergoes deprotection by a heat treatment in a triethylamine acetate (TEAA) buffer solution containing 45-50 v/v % formamide at a pH of 8-9 or in a phosphate buffer solution containing 40 to 60 v/v % dimethylsulfoxide (DMSO) at a pH of 7.1-8.

In certain embodiments, the heat treatment comprises:
a) treating the product obtained in step (2) at a temperature of 80 to 95°C for 5 to 10 minutes, or,
b) treating the product obtained in step (2) at a temperature of 60 to 70°C for 1.5 to 2.5 hours (e.g., about 2h).

In certain embodiments, the product obtained in step (2) undergoes deprotection by the heat treatment as described in a) in a triethylamine acetate (TEAA) buffer solution containing about 47.5 v/v% formamide at a pH of about 8.6; or,
the product obtained in step (2) undergoes deprotection by the heat treatment as described in b) in a phosphate buffer solution containing about 50 v/v % dimethylsulfoxide (DMSO) at a pH of about 7.4.

In certain embodiments, in the method, the nucleic acid molecule to be detected is RNA or a DNA/RNA hybrid.

In certain embodiments, in step (3), the product obtained in step (2) undergoes deprotection under an alkaline condition.

In some embodiments, in step (3), the product obtained in step (2) undergoes a first deprotection under an alkaline condition, and the product obtained by the first deprotection undergoes a second deprotection in a phosphate buffer solution.

In certain embodiments, in step (3), the product obtained in step (2) undergoes deprotection by a heat treatment under an alkaline condition.

In some embodiments, in step (3), the product obtained in step (2) undergoes a first deprotection by heat treatment in a triethylamine acetate (TEAA) buffer solution containing formamide at a pH of 8 to 9.

In some embodiments, in step (3), the product obtained in step (2) undergoes a first deprotection by a heat treatment in a triethylamine acetate (TEAA) buffer solution containing formamide at a pH of 8 to 9, and the product obtained by the first deprotection undergoes a second protection by a heat treatment in a phosphate buffer solution containing dimethylsulfoxide (DMSO) at a pH of 7.1 to 8.

In some embodiments, in step (3), the product obtained in step (2) undergoes a first deprotection by a heat treatment in a triethylamine acetate (TEAA) buffer solution containing 45 to 50 v/v% formamide at a pH of 8 to 9.

In some embodiments, in step (3), the product obtained in step (2) undergoes a first deprotection by a heat treatment in a triethylamine acetate (TEAA) buffer solution containing 45 to 50 v/v% formamide at a pH of 8 to 9, and the product obtained by the first deprotection undergoes a second protection by a heat treatment in a phosphate buffer solution containing 40 to 60 v/v % dimethylsulfoxide (DMSO) at a pH of 7.1 to 8.

In certain embodiments, the heat treatment comprises:
a) treating the product obtained in step (2) at a temperature of 80 to 95°C for 5 to 10 minutes, or,
b) treating the product obtained in step (2) at a temperature of 60 to 70°C for 1.5 to 2.5 hours (e.g., about 2h).

In certain embodiments, in step (3), the product obtained in step (2) undergoes a first deprotection by the heat treatment as described in a) in a triethylamine acetate (TEAA) buffer solution containing 47.5 v/v% formamide at a pH of about 8.6.

In certain embodiments, in step (3), the product obtained in step (2) undergoes a first deprotection by the heat treatment as described in a) in a triethylamine acetate (TEAA) buffer solution containing 47.5 v/v% formamide at a pH of about 8.6, and the product obtained by the first deprotection undergoes a second protection by the heat treatment as described in b) in a phosphate buffer solution containing 50 v/v % dimethylsulfoxide (DMSO) at a pH of about 7.4.

In certain embodiments, in the method, the nucleic acid molecule to be detected is a DNA or a DNA/RNA hybrid.

In certain embodiments, in step (3), the product obtained in step (2) undergoes deprotection under an alkaline condition.

In certain embodiments, in step (3), the product obtained in step (2) undergoes deprotection by a heat treatment under an alkaline condition.

In certain embodiments, the product obtained in step (2) undergoes deprotection by a heat treatment in a triethylamine acetate (TEAA) buffer solution containing formamide at a pH of 8 to 9.

In certain embodiments, the product obtained in step (2) undergoes deprotection by a heat treatment in a triethylamine acetate (TEAA) buffer solution containing 45 to 50 v/v % formamide at a pH of 8 to 9.

In some embodiments, the heat treatment comprises: treating the product obtained in step (2) at a temperature of 80 to 95°C for 5 to 10 minutes.

In certain embodiments, the product obtained in step (2) undergoes the heat treatment as described in a) in a triethylamine acetate (TEAA) buffer solution containing about 47.5 v/v% formamide at a pH of about 8.6.

In one aspect, the present application also provides a kit, which comprises a first carbonyl compound and a nitrite.

In certain embodiments, the first carbonyl compound is selected from the group consisting of compounds represented by Formula I and any combination thereof, 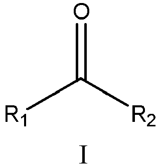
wherein R₁ is an aldehyde group, C₁₋₃ alkyl group (e.g., methyl, ethyl or n-propyl), or halogen-substituted C₁₋₃ alkyl (e.g., trichloromethyl, trifluoromethyl), R₂ is H, C₁₋₃ alkyl (e.g., methyl, ethyl or n-propyl), phenyl or HC(=O)-CH(Br)-; or,
R₁ and R₂ together with the carbonyl to which they are bonded form a cyclic structure, such as ninhydrin.

In certain embodiments, the first carbonyl compound is selected from the group consisting of: glyoxal, 2,3-butanedione, ninhydrin, 2-bromomalondialdehyde, pyruvaldehyde, trichloroacetaldehyde, phenylglyoxal, and any combination thereof.

In certain embodiments, the first carbonyl compound is selected from the group consisting of: glyoxal, 2,3-butanedione, ninhydrin, 2-bromomalondialdehyde, pyruvaldehyde, trichloroacetaldehyde, phenylglyoxal, and any combination thereof.

In certain embodiments, the first carbonyl compound is selected from the group consisting of glyoxal, 2-bromomalondialdehyde, 2,3-butanedione, phenylglyoxal, trichloroacetaldehyde, ninhydrin, and any combination thereof.

In certain embodiments, the kit further comprises a second carbonyl compound.

In certain embodiments, the second carbonyl compound is selected from the group consisting of compounds represented by Formula I and any combination thereof, 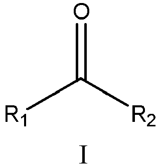
wherein R₁ is an aldehyde, C₁₋₃ alkyl (e.g., methyl, ethyl or n-propyl), or halogen-substituted C₁₋₃ alkyl (e.g., trichloromethyl, trifluoromethyl), R₂ is H, C₁₋₃ alkyl (e.g., methyl, ethyl or n-propyl), phenyl or HC(=O)-CH(Br)-; or,
R₁ and R₂ together with the carbonyl to which they are bonded form a cyclic structure, such as ninhydrin.

In certain embodiments, the second carbonyl compound is selected from the group consisting of: glyoxal, ninhydrin, and any combination thereof.

In certain embodiments, the nitrite is selected from the group consisting of sodium nitrite, potassium nitrite, and combinations thereof.

In certain embodiments, the kit further comprises boric acid or salt thereof (e.g., potassium borate).

In certain embodiments, the kit further comprises dimethylsulfoxide (DMSO), N,N-dimethylformamide (DMF), or a combination thereof.

In certain embodiments, the kit further comprises at least one substance selected from the group consisting of: 2-(N-morpholino)ethanesulfonic acid (MES), sodium acetate, 3-(N-morpholino)propanesulfonic acid (MOPS), piperazine-1,4-diethanesulfonic acid (PIPES), 4-hydroxyethylpiperazineethanesulfonic acid (HEPPS), and tris(hydroxymethyl)aminomethane (TRIS); or the kit further comprises p-toluenesulfonic acid, phosphoric acid, or a combination thereof.

In certain embodiments, the kit further comprises at least one substance selected from the group consisting of p-toluenesulfonic acid, phosphoric acid, 2-(N-morpholino)ethanesulfonic acid (MES), sodium acetate, 3-(N-morpholino)propanesulfonic acid (MOPS), piperazine-1,4-diethanesulfonic acid (PIPES), 4-hydroxyethylpiperazineethanesulfonic acid (HEPPS), and tris(hydroxymethyl)aminomethane (TRIS).

In certain embodiments, the kit further comprises a substance selected from the group consisting of: a substance for preparing a 2-(N-morpholino)ethanesulfonic acid (MES) buffer solution, a substance for preparing a sodium acetate buffer solution, a substance for preparing a 3-(N-morpholino)propanesulfonic acid (MOPS) buffer solution, a substance for preparing a piperazine-1,4-diethanesulfonic acid (PIPES) buffer solution, a substance for preparing a 4-hydroxyethylpiperazinethanesulfonic acid (HEPPS) buffer solution, a substance for preparing a tris(hydroxymethyl)aminomethane (TRIS) buffer solution, and any combination thereof.

In certain embodiments, the kit further comprises a 2-(N-morpholino)ethanesulfonic acid (MES) buffer solution, a sodium acetate buffer solution, a 3-(N-morpholino)propanesulfonic acid (MOPS) buffer solution solution, a piperazine-1,4-diethanesulfonic acid (PIPES) buffer solution, a 4-hydroxyethylpiperazineethanesulfonic acid (HEPPS) buffer solution, or a tris(hydroxymethyl)aminomethane (TRIS) buffer solution, or any combination thereof.

In certain embodiments, the kit further comprises a substance selected from the group consisting of a substance for preparing a phosphate buffer solution, a substance for preparing a triethylamine acetate (TEAA) buffer solution, and a combination thereof.

In certain embodiments, the kit further comprises a phosphate buffer solution, a triethylamine acetate (TEAA) buffer solution, or a combination thereof.

The substances for preparing the buffer solutions described in the present application may exist in dry powder form or in solution form. The substances for preparing the buffer solutions are well known in the art and can be selected according to experimental needs and conventional methods in the art.

For example, the substance for preparing the 2-(N-morpholino)ethanesulfonic acid (MES) buffer solution comprises 2-morpholinoethanesulfonic acid, and optionally sodium hydroxide. The substance for preparing the sodium acetate buffer solution comprises sodium acetate, and optionally acetic acid. The substance for preparing the 3-(N-morpholino)propanesulfonic acid (MOPS) buffer solution comprises 3-(N-morpholino)propanesulfonic acid, and optionally sodium hydroxide. The substance for preparing the piperazine-1,4-diethanesulfonic acid (PIPES) buffer solution comprises piperazine-1,4-diethanesulfonic acid, and optionally sodium hydroxide. The substance for preparing the 4-hydroxyethylpiperazinethanesulfonic acid (HEPPS) buffer solution comprises 4-hydroxyethylpiperazineethanesulfonic acid, and optionally sodium hydroxide. The substance for preparing the tris(hydroxymethyl)aminomethane buffer solution comprises tris(hydroxymethyl)aminomethane, and optionally hydrochloric acid.

In certain embodiments, the triethylamine acetate (TEAA) buffer solution comprises 45 to 50 v/v % formamide and has a pH of 8 to 9.

In certain embodiments, the phosphate buffer solution comprises 40 to 60 v/v % dimethylsulfoxide (DMSO) and has a pH of 7.1 to 8.

In certain embodiments, the kit further comprises a reagent for mass spectrometry detection and/or DNA sequencing.

In certain embodiments, the kit further comprises an instruction.

In certain embodiments, the instruction describes a method as described above.

In one aspect, the present application also provides a use of the kit as described above in the detection of N6-methyladenine in a nucleic acid molecule.

### Definition of Terms

In the present application, unless otherwise stated, scientific and technical terms used herein have the meanings commonly understood by those skilled in the art. Meanwhile, in order to better understand the present application, definitions and explanations of relevant terms are provided below.

When the terms "for example," "e.g.," "such as," "comprising," "including," or variations thereof are used herein, these terms will not be considered limiting terms and will instead be interpreted to mean "but not being limited" or "without limitation."

Unless otherwise indicated herein or clearly contradicted by context, the terms "a" and "an" as well as "the" and similar referents in the context of describing the present application (especially in the context of the following claims) are to be construed to cover singular and plural forms.

As used herein, unless the context clearly indicates otherwise, the term "adenine" or "A" as used herein encompasses adenine itself, as well as adenine-containing nucleotides (e.g., ribonucleotides, deoxyribonucleotides, adenine ribonucleotide residues, adenine deoxyribonucleotide residues); the term "N6-methyladenine", "6mA", "m6A" or "m⁶A" encompasses N6-methyladenine itself, as well as nucleotides containing N6-methyladenine (e.g., ribonucleotides, deoxyribonucleotides, ribonucleotide residues, deoxyribonucleotide residues); the term "hypoxanthine" or "I" encompasses hypoxanthine itself, as well as hypoxanthine-containing nucleotides (e.g., ribonucleotides, deoxyribonucleotides, ribonucleotide residues, deoxyribonucleotide residues); the term "xanthine" or "X" encompasses xanthine itself, as well as xanthine-containing nucleotides (e.g., ribonucleotides, deoxyribonucleotides, ribonucleotide residues, deoxyribonucleotide residues); the term "guanine" or "G" encompasses guanine itself, as well as guanine-containing nucleotides (e.g., ribonucleotides, deoxyribonucleotides, ribonucleotide residues, deoxyribonucleotide residue); the term "thymine" or "T" encompasses thymine itself, as well as thymine-containing nucleotides (e.g., ribonucleotides, deoxyribonucleotides, ribonucleotide residues, deoxyribonucleotide residues); the term "cytosine" or "C" encompasses cytosine itself, as well as cytosine-containing nucleotides (e.g., ribonucleotides, deoxyribonucleotides, ribonucleotide residues, deoxyribonucleotide residues); the term "uracil" or "U" encompasses uracil itself, as well as uracil-containing nucleotides (e.g., ribonucleotides, deoxyribonucleotides, ribonucleotide residues, deoxyribonucleotide residues).

As used herein, unless the context clearly indicates otherwise, the terms "m6A", "6mA" and "m⁶A" as used herein encompass N6-methyladenine itself in DNA, RNA or DNA/RNA hybrid, and N6-methyladenine-containing nucleotides (e.g., ribonucleotides, deoxyribonucleotides, ribonucleotide residues, deoxyribonucleotide residues), and are used interchangeably.

As used herein, unless the context clearly indicates otherwise, the compounds used herein have the meanings commonly understood by those skilled in the art. For example, the compounds used herein and their corresponding CAS registry numbers are as follows: 2-(N-morpholino)ethanesulfonic acid (MES, CAS: 4432-31-9), sodium acetate (CAS: 127-09-3), 3-(N-morpholino)propanesulfonic acid (MOPS, CAS: 1132-61-2), piperazine-1,4-diethanesulfonic acid (PIPES, CAS: 5625-37-6), 4-hydroxyethylpiperazineethanesulfonic acid (HEPPS, CAS: 7365-45-9) or tris(hydroxymethyl)aminomethane (TRIS, CAS: 77-86-1), glyoxal (CAS: 107-22-2), pyruvaldehyde (CAS: 78-98-8), 2,3-butanedione (CAS: 431-03-8), ninhydrin (CAS: 485-47-2), 2-bromomalondialdehyde (CAS: 2065-75-0), trichloroacetaldehyde (CAS: 75-87-6), phenylglyoxal (CAS: 1075-06-5), formamide (CAS: 75-12-7), p-toluenesulfonic acid (CAS: 104-15-4), phosphoric acid (CAS: 7664-38-2), sodium hydroxide (CAS: 1310-73-2), hydrochloric acid (CAS: 7647-01-0), acetic acid (CAS: 64-19-7).

As used herein, various buffer solutions used herein have meanings commonly understood by those skilled in the art, and can be prepared according to conventional methods in the art according to experimental needs. For example, the 2-(N-morpholino)ethanesulfonic acid (MES) buffer solution as described herein can be prepared with 2-(N-morpholino)ethanesulfonic acid (MES), optionally with sodium hydroxide solution to adjust pH as needed. For example, the sodium acetate buffer solution used herein can be prepared with sodium acetate, optionally with acetic acid to adjust pH as needed. For example, the 3-(N-morpholino)propanesulfonic acid (MOPS) buffer solution used herein can be prepared with 3-(N-morpholino)propanesulfonic acid, optionally with sodium hydroxide to adjust pH as needed. For example, the piperazine-1,4-diethanesulfonic acid (PIPES) buffer solution used herein can be prepared with piperazine-1,4-diethanesulfonic acid, optionally with sodium hydroxide to adjust pH as needed. For example, the 4-hydroxyethylpiperazine sulfonic acid (HEPPS) buffer solution used herein can be prepared with 4-hydroxyethylpiperazine sulfonic acid, optionally with sodium hydroxide to adjust pH as needed. For example, the tris(hydroxymethyl)aminomethane (TRIS) buffer solution used herein can be prepared with tris(hydroxymethyl)aminomethane, optionally with hydrochloric acid to adjust pH as needed.

### Beneficial Effects

Compared with the technology in the prior art, the technical solution of the present application has the following beneficial effects:
(1) By using the method of the present application, the A-to-I conversion rate is high, thereby enabling quantitative and/or qualitative detection of low-abundance m⁶A sites.
(2) By using the method of the present application, the conversion rate of false positive or non-specific conversion (e.g., C-to-U conversion, G to X conversion) is low, and the background signal is low when m⁶A is analyzed.
(3) In the method of the present application, a nitrite is used to catalyze the deamination of adenine into hypoxanthine, while N6-methyladenine cannot be deaminated due to its stable chemical properties. Therefore, the method of the present application achieves detection of m⁶A sites at single-base resolution by analyzing the signals of adenine or hypoxanthine before and after conversion.

Therefore, the present application provides a highly active and specific m⁶A site detection technology, which can quantitatively analyze m⁶A sites in the whole genome or the whole transcriptome at single-base resolution, solving the problems of traditional detection methods.

The embodiments of the present application will be described in detail below in conjunction with the drawings and examples, but the those skilled in the art will understand that the following drawings and examples are only used to illustrate the present application and do not limit the scope of the present application. Various objects and advantageous aspects of the present application will become apparent to those skilled in the art from the accompanying drawings and the following detailed description of preferred embodiments.

### Brief Description of the Drawings

Fig. 1 schematically shows the reaction process of the conversion of adenine to hypoxanthine under the catalysis of a nitrite and a carbonyl compound.
Fig. 2 schematically shows, for example, when a dicarbonyl compound is used to protect guanine, the reaction process of forming a guanosine adduct by the guanine base and the dicarbonyl compound.
Fig. 3 schematically shows the reaction process of forming a new guanosine adduct by a boric acid compound and a guanosine adduct.
Fig. 4 shows the Sanger sequencing peak map of the amplified product of the DNA model after being treated with reaction conditions A, B or C.
Fig. 5 shows the Sanger sequencing peak map of the amplified products of the DNA model before and after treatment with each reaction condition in Table 3.
Fig. 6 shows the Sanger sequencing peak map of the amplified products of the DNA model before and after treatment with each reaction condition in Table 4.
Fig. 7 shows the Sanger sequencing peak map of the amplified products of the DNA model before and after treatment with each reaction condition in Table 5.
Fig. 8 shows the Sanger sequencing peak map of the amplified products of the DNA model before and after treatment with each reaction condition in Table 6.
Fig. 9 shows the Sanger sequencing peak map of the amplified products of the DNA model before and after treatment with each reaction condition in Table 7.
Fig. 10 shows the Sanger sequencing peak map of the amplified products of the DNA model before and after treatment with each reaction condition in Table 8.
Fig. 11 shows the Sanger sequencing peak map of the amplified products of the DNA model before and after treatment with each reaction condition in Table 9.
Fig. 12 shows the Sanger sequencing peak map of the amplified products of the DNA model before and after treatment with each reaction condition in Table 10.
Fig. 13 shows the Sanger sequencing peak map of the amplification products of the DNA model before and after treatment with each reaction condition in Table 11.
Fig. 14 shows the Sanger sequencing peak map of the amplification products of the DNA model before and after treatment with each reaction condition in Table 12.
Fig. 15 shows the Sanger sequencing peak map of the amplification products of the DNA model before and after treatment with each reaction condition in Table 13.
Fig. 16 shows the Sanger sequencing peak map of the amplification products of the DNA model before and after treatment with each reaction condition in Table 14.
Fig. 17 shows the Sanger sequencing peak map of the amplification products of the DNA model before and after treatment with each reaction condition in Table 15.
Fig. 18 shows the Sanger sequencing peak map of the amplification products of the DNA model before and after treatment with each reaction condition in Table 16.
Fig. 19 shows the Sanger sequencing peak map of the amplification products of the DNA model before and after treatment with each reaction condition in Table 17.
Fig. 20 shows the A-to-G signal conversion efficiency in RNA samples achieved by the method of the present application. (a) shows, in the sequencing data, the signal conversion rates of A-to-G in rRNA and model sequences; among them, m6A-1 and m6A-2 represent two biological replicates. (b) shows, in the sequencing data, the signal conversion rate of A-to-G in mRNA.
Fig. 21 shows the C-to-T signal conversion efficiency in RNA samples achieved by the method of the present application. (a) shows, in the sequencing data, the signal conversion rates of C-to-T in rRNA and model sequences; among them, m6A-1 and m6A-2 represent two biological replicates. (b) shows, in the sequencing data, the signal conversion rate of C-to-T in mRNA.

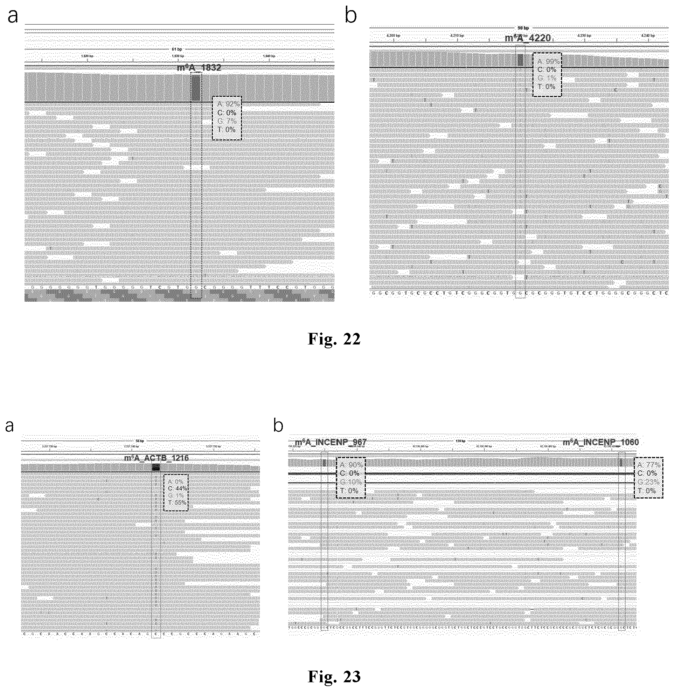

Fig. 22 shows the high-throughput sequencing signal on rRNA by the method of the present application. (a) shows the signal of the classic m⁶A_1832 site on 18S rRNA during sequencing. In the red rectangle, there is the m⁶A modification site, and in the rectangle with a black border, provided are percentages of the reads covering the site that is read as A, T, C, or G bases, respectively; (b) shows the signal of the classic m⁶A_4220 site on 28S rRNA during sequencing. The reference transcriptome (version number GRCh38.p13; GCF _000001405.39) with A converted into G is used as an alignment template, and the green block shows the G-to-A mutation signal generated in the sequencing signal as compared to the alignment template, indicating that most reads have base A at this position.
Fig. 23 shows the high-throughput sequencing signal on mRNA by the method of the present application. (a) shows the signal of m⁶A site 1216 (chr7:5527743, version hg38) on the mRNA of gene ACTB during sequencing. In the red rectangle, there is the m⁶A modification site, and in the rectangle with a black border, provided are percentages of the reads covering the site that is read as A, T, C, or G bases, respectively. The negative strand of the reference transcriptome (version number GRCh38.p13; GCF _000001405.39) with T converted into C is used as an alignment template, and the m⁶A modification in the negative strand transcribed mRNA shows a C-to-T mutation signal as compared to the alignment template during sequencing. (b) shows the signals of m⁶A sites 967 and 1060 (chr11:62130330; chr11:62130423) on the mRNA of gene INCENP during sequencing. INCENP is a positive strand gene. The reference transcriptome (version number GRCh38.p13; GCF _000001405.39) with A converted into G is used as an alignment template, and the green block in the panel shows the G-to-A mutation signal generated in the sequencing signal as compared to the alignment template., indicating that most reads have base A at the indicated position.
Fig. 24 shows the distribution of m⁶A in mRNA detected by the method of the present application.

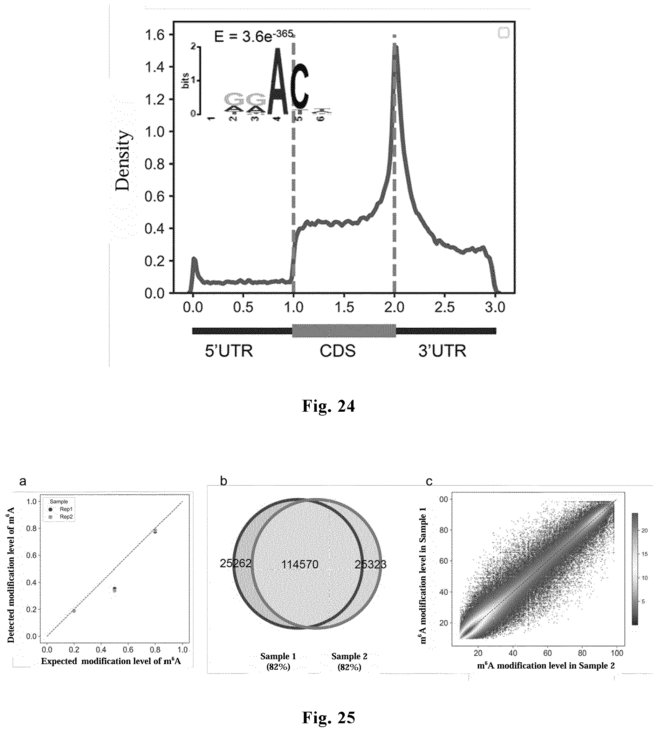

Fig. 25 shows the m⁶A modification levels in model sequences and mRNA detected by the method of the present application. (a) shows comparison of the m⁶A modification level as detected in the model sequence of the present application, with the expected modification level of the model sequence as used, in which Rep-1 and Rep-2 represent biological samples in duplicate. (b) shows the number and repeatability of m⁶A detected by the method of the present application in HEK293T cell mRNA in duplicate. (c) shows the comparison of the modification levels of the corresponding m⁶A modification sites detected by the method of the present application in HEK293T cell mRNA in duplicate.
Fig. 26 shows the A-to-G signal conversion efficiency in mRNA samples detected by the method of the present application under different reaction conditions. (a) shows, in the sequencing data, the A-to-G signal conversion rates of mRNA under different reaction conditions; (b) shows the comparison of the m⁶A modification level detected in the model sequence by the method of the present application under different reaction conditions with the expected modification level of the model sequence. In the figure, "NS-16" corresponds to the exemplary conversion reaction condition C in Example 3, "NS-50" corresponds to the exemplary conversion reaction condition D in Example 3, and "NS-TsOH" corresponds to the exemplary conversion reaction condition E in Example 3.
Fig. 27 shows the secondary mass spectrum of the imine intermediate produced during the reaction of using glyoxal to catalyze the conversion of adenine ribonucleotide into hypoxanthine ribonucleotide in the method of the present application.

### Sequence Information

**Table 1: Information of the sequences covered by the present application is described in the table below.**

| SE Q ID NO: | Description of sequence |
|---|---|
| 1 | Sequence of DNA model: |
| | GTGTGTTGTGTGTGTTTGTGCG6mAGTAACTGACCAGATCTCTATACGTG ACTCATGATGGCTTTGGTTGGTTGGTTTGG |
| 2 | Sequence of DNA amplification primer 1: |
| | AACTGGAGAGCATCCTGCTGTTGGTCCACATGGCTAGTGATGGCTACTG AGTCAGGTCCATTGATGCACCAAACCAACCAACCAAA |
| 3 | Sequence of DNA amplification primer 2: |
| | GTGTGTTGTGTGTGTTTGTG |
| 4 | Primer for DNA sequencing: |
| | AACTGGAGAGCATCCTGCTG |
| 5 | Sequence of 3'RNA linker |
| | rAPP-AGATCGGAAGAGCGTCGTG-3 SpC3 |
| 6 | Sequence of 5'adaptor |
| | 5Phos-NNNNNNNNNNAGATCGGAAGAGCACACGTCTG-3SpC3 |
| 7 | Sequence of cDNA amplification primer 1 |
| | AATGATACGGCGACCACCGAGATCTACACTCTTTCCCTACACGACGCTCT TCCGATCT |
| 8 | Sequence of cDNA amplification primer 2 |
| | CAAGCAGAAGACGGCATACGAGATXXXXXXGTGACTGGAGTTCAGACG TGTGCTCTTCCGATC |
| 9 | Nucleotide sequence of Spike in 1 |
| | GUUGAUCUUGGCACGUCUACUCGACCGUCUGGAACUUGAUAUUAUCG GAUCAGUGAAUCACUCCAAGCUGACAG |
| 10 | Nucleotide sequence of Spike in 2 |
| | AGCGCAGCGAACAUGACACGUGCUCAACAGUGUAGUUGGm6ACUUCCU CGCAUCUAGCCGCUAUGCACGUGCAGCU |
| 11 | Nucleotide sequence of Spike in 2' |
| | AGCGCAGCGAACAUGACACGUGCUCAACAGUGUAGUUGGACUUCCUCG CAUCUAGCCGCUAUGCACGUGCAGCU |
| 12 | Nucleotide sequence of Spike in 3 |
| | GAUCGUAGCAUUGGUUAGCGUGUUGGUGGAUGUAGUGAm6ACUAAUC GCCUUAUAGGUGCCAAAUGUCAUGGACAGC |
| 13 | Nucleotide sequence of Spike in 3' |
| | GAUCGUAGCAUUGGUUAGCGUGUUGGUGGAUGUAGUGAACUAAUCGC CUUAUAGGUGCCAAAUGUCAUGGACAGC |
| 14 | Nucleotide sequence of Spike in 4 |
| | AUCUACCUGUCCAGUAGCCUUCAGGAUCAUGCUGUCUGACUUGCUGGm 6ACAUCAUUCUAGUGCCAUAACUUCAGC |
| 15 | Nucleotide sequence of Spike in 4' |
| | AUCUACCUGUCCAGUAGCCUUCAGGAUCAUGCUGUCUGACUUGCUGGA CAUCAUUCUAGUGCCAUAACUUCAGC |
| 16 | Nucleotide sequence of Spike in 5 |
| | UAUGGUUUAGAUUGAAUGUUGUUGUUGUGAGGm⁶ACCUUAUGUAAGU UGAAUGGUAAUAGGAAGAAGUUUG |
| 17 | Nucleotide sequence of Spike in 5' |
| | UAUGGUUUAGAUUGAAUGUUGUUGUGAGGACCUUAUGUAAGUUG AAUGGUAAUAGGAAGAAGUUUG |
| 18 | Nucleotide sequence of Spike in 6 |
| | UUGUUUGAUGGAGGGGGAUAAUUAUUGGAm⁶ACAGGUAGUUAAUAUU GUAUAAUGUUGUAAGAUUAAAGAGGG |
| 19 | Nucleotide sequence of Spike in 6' |
| | UUGUUUGAUGGAGGGGGAUAAUUAUUGGAACAGGUAGUUAAUAUUGU AUAAUGUUGUAAGAUUAAAGAGGG |
| 20 | Nucleotide sequence of Spike in 7 |
| | GGAUUAGUUAGUAGGUGGGUAAUGGUUUAUGAm6ACCUGAUGAUUU UUAGUUGGUUUGAGAGGAUGAUUAGU |

| | |
|---|---|
| Note: "N"=A, T, C or G; "X"=A, T, C or G; | |

### Specific Models for Carrying Out the Invention

The present application will now be described with reference to the following examples which are intended to illustrate, rather than limit, the present application.

Those skilled in the art will appreciate that the examples describe the present application by way of example and are not intended to limit the scope sought to be protected by the present application. The experimental methods in the examples are all conventional methods unless otherwise specified. If the specific conditions are not specified in the examples, the experimental methods should be carried out according to the conventional conditions or the conditions recommended by the manufacturer. If the manufacturer of the reagents or instruments used is not indicated, they are all conventional products that can be purchased commercially.

### Preparation of solutions:

Deprotection A buffer: 0.5 M triethylamine acetate (TEAA), pH 8.6, 47.5% formamide;
Deprotection B buffer: 50% dimethylsulfoxide (DMSO) in 137 mM NaCl, 2.7 mM KCl, 8 mM Na₂HPO₄, 2 mM KH₂PO₄, pH 7.4;
5 M NaNO₂: 345mg of NaNO₂ was dissolved in DEPC water and replenished with water to 1ml;
500 mM MES buffer (pH 5): 106.6 mg of 2-(N-morpholino)ethanesulfonic acid (MES) was dissolved in 900 µL of DEPC water, adjusted to pH 5 with 1 M NaOH, and replenished with water to 1 mL;
500 mM MES buffer (pH 5.5): 106.6 mg of MES was dissolved in 900 µL of DEPC water, adjusted to pH 5.5 with 1 M NaOH, and replenished with water to 1 mL;
500 mM MES buffer (pH 6): 106.6 mg of MES was dissolved in 800 µL of DEPC water, adjusted to pH 6 with 1 M NaOH, and replenished with water to 1 mL;
H₃BO₃ buffer: 100 mg of H₃BO₃ was taken and added to 1 mL of DEPC water, mixed well, and the supernatant was taken for later use (H₃BO₃ saturated solution);
p-toluenesulfonic acid aqueous solution containing 5 M NaNO₂ (pH 6): 345mg of NaNO₂ was dissolved in DEPC water, adjusted with p-toluenesulfonic acid to pH 6, and replenished with water to 1ml;
2-Bromomalondialdehyde solution: it was a DMSO/H₂O (1:1) solution containing 2.0 M 2-bromomalondialdehyde, 301.9 mg of 2-bromomalondialdehyde was taken and dissolved in 0.5 mL of dimethylsulfoxide (DMSO), mixed well, then added with 0.5 mL of DEPC water, and mixed well;
Ninhydrin solution: it was a 0.15 M of ninhydrin solution, 26.7 mg of ninhydrin was taken, dissolved in 1 mL of DMSO, and mixed well;
75% ethanol: 30mL of absolute ethanol was added to 10mL of DEPC water and mixed well;
1 M NH₄OAc (pH 5.3): 77.0 mg of ammonium acetate was taken and dissolved in 800 µL of DEPC water, adjusted to pH 5.3 with acetic acid, and replenished with water to 1 mL.

### Sources of reagents:

NaNO₂, 40% glyoxal aqueous solution (molar concentration: 8.8 M), phenylglyoxal, 2-bromomalondialdehyde, trichloroacetaldehyde, anhydrous sodium acetate and other chemical reagents were purchased from Energy Chemical;
MES was purchased from Amresco;
p-Toluenesulfonic acid was purchased from Energy Chemical;
H₃BO₃ was purchased from Aladdin;
DEPC water is purchased from Sangon Biotech;
DMSO, absolute ethanol and other solvents were purchased from Beijing Tong Guang Fine Chemical Company;
The amplification enzyme used for PCR was KOD One^{™} PCR Master Mix, which was purchased from TOYOBO;
Nucleic acid precipitation aid (Glycogen) was purchased from ZOMANBIO;
Total RNA was extracted from HEK293T cells using an RNA extraction kit (Trizol method), and the kit was purchased from Invitrogen, Cat. No.: 15596026;
Nuclease P1 (Nuclease P1 from Penicillium citrinum lyophilized powder) was purchased from SIGMA;
Alkaline phosphatase (Shrimp Alkaline Phosphatase) rSAP was purchased from SIGMA;
The DNA model sequence shown in SEQ ID NOs: 1 was synthesized using a DNA synthesizer (ABI 3400), and the synthesis specification was 1 umol. Nucleic acid monomers (A, T, C, G and 6mA) were purchased from Anhui Wuhu Huaren Technology Co., Ltd.;
Bacterial genomic DNA extraction kit was purchased from Tiangen, Cat. No.: DP302;
DNA uracil glycosidase was purchased from New England Biolabs (NEB) (Cat. No.: M0280).

After the detection, it was found that the reagents from various sources had no significant impact on the experimental results, and the conditions were stable.

### Example 1: Detection of 6mA in DNA model

### I. Experimental method

### 1. Conversion reaction of A (adenine) to I (hypoxanthine)

The conversion reaction of A-to-I could be carried out with reference to the exemplary reaction conditions A, B or C.

### Exemplary Reaction Conditions A for conversion reaction

### 1.1 Protection of nucleic acid

1µg of 6mA DNA model (the sequence was shown in SEQ ID NO: 1) was taken and dissolved in 2.8 µL of DEPC water, added with 4 µL of DMSO and 1.2 µL of 40 wt/wt% glyoxal aqueous solution (the final concentration of glyoxal was approximately 1.3 M), and mixed well, then heated to 50°C and incubated for 1 hour to obtain a nucleic acid protected by glyoxal, which was then cooled on ice for later use.

### 1.2 Nitrosation reaction

Preparation of reaction solution: 3 µL of 5 M NaNO₂, 1.6 µL of 500 mM MES (pH 6), 2 µL of H₃BO₃, and 5.4 µL of DEPC water were added with 8 µL of the nucleic acid protected by glyoxal prepared in step 1.1 above, to a total volume of 20 µL, and the reaction was carried out at 16°C for 24 h.

### Exemplary Reaction Conditions B for conversion reaction

### 1.1 Protection of nucleic acid

1µg of 6mA DNA model (the sequence was shown in SEQ ID NO: 1) was taken and dissolved in 2.8 µL of DEPC water, added with 4 µL of DMSO and 1.2 µL of 40% glyoxal, and mixed well, then heated to 50°C for 1 hour to obtain a nucleic acid protected by glyoxal, which was then cooled on ice for later use.

### 1.2 Nitrosation reaction

Preparation of reaction solution: 3 µL of 5 M NaNO₂, 1.6 µL of 500 mM MES (pH 6), 2 µL of H₃BO₃, 3.4 µL of DEPC water, and 2 µL of 40% glyoxal aqueous solution were added with 8 µL of the nucleic acid protected by glyoxal prepared in step 1.1 above, to a total volume of 20 µL, and the reaction was carried out at 16°C for 8 h.

### Exemplary Reaction Conditions C for conversion reaction

### 1.1 Protection of nucleic acid

1µg of 6mA DNA model (the sequence was shown in SEQ ID NO: 1) was taken and dissolved in 2.8 µL of DEPC water, added with 4 µL of DMSO and 1.2 µL of 40% glyoxal, and mixed well, then heated at 50°C for 0.5 h; subsequently, 2 µL of H₃BO₃ was added to the mixture, incubated at 50°C for 0.5 hours, to obtain a nucleic acid protected by glyoxal, which was then cooled on ice for later use.

### 1.2 Nitrosation reaction

Preparation of reaction solution: 3 µL of 5 M NaNO₂, 1.6 µL of 500 mM MES (pH 6), 3.4 µL of DEPC water, and 2 µL of 40% glyoxal aqueous solution were added with 10 µL of the nucleic acid protected by glyoxal prepared in step 1.1 above, to a total volume of 20 µL, and the reaction was carried out at 16°C for 8 h.

### 2. Purification of conversion product

2.1 To the sample obtained from the conversion reaction in step 1 above, 2 µL of 3 M NaOAc (pH 5.2), 2 µL of Glycogen, and 55 µL of 75% ethanol (the final concentration of ethanol was about 70%) were added and mixed well, allowed to stand at -80°C for 2 hours, then centrifuged at 12,000 g for 10 minutes, added with 1 ml of 70% ethanol and centrifuged again to remove the liquid.

2.2 To the precipitate obtained in step 2.1 above, 50 µL of Deprotection A buffer was added, reacted at 95°C for 10 minutes, placed on ice, added with 5 µL of 3 M NaOAc (pH 5.2), 2 µL of Glycogen and mixed well, then added with 142 µL of absolute ethanol, allowed to stand at -80°C, 2h, centrifuged at 12,000 g for 10 minutes, added with 1 mL of 70% ethanol and centrifuged again to remove the liquid.

2.3 The DNA precipitate prepared in step 2.2 above was dissolved in 50 µL of DEPC water and placed on ice for later use.

### 3. Sanger sequencing

3.1 To a 0.2 ml PCR tube, 15 µL of KOD One^{™} PCR Master Mix, 11 µL of ddH₂O, 1 µL of the DNA sample prepared in step 2 above as a template, 1.5 µL of amplification primer 1 (SEQ ID NO: 2), and 1.5 µL of amplification primer 2 (SEQ ID NO: 3) were added, then amplification was carried out for 35 cycles for later use.

3.2 Since I (hypoxanthine) converted from A (adenine) in the DNA sequence complementarily paired with C (cytosine) during the DNA amplification process, it was read as G (guanine), and the 6mA in the sequence was not converted to I, and remained complementary pairing to T (thymine), and was read as A, then the conversion efficiency of A-to-I and the position information of 6mA in the original model sequence could be determined by sequencing the amplification product obtained in step 3.1 using Sanger method, and by comparing the sequence information of the amplification product with the original DNA model sequence. The primer sequence for sequencing was shown in SEQ ID NO: 4.

### 4. Triple tandem quadrupole mass spectrometry (QQQ) detection

200 ng of the DNA sample prepared in step 2 above was added with 1U of nuclease P1, 2 µL of NH₄OAc (pH=5.3), replenished with water to 20 µL, and reacted at 42°C for 2 hours; then added with 1 µL of FastAP Thermosensitive Alkaline Phosphatase, 2.5 µL of 10× FastAP Buffer, replenished with water to 25 µL, and reacted at 37°C for 2 hours.

The solution after the above reaction was centrifuged at 4°C, 12000 r, for 10 minutes, then 25 µL thereof was taken for QQQ detection to measure the content of I in the sample DNA undergoing the nitrosation reaction, thereby further determining the conversion efficiency of A-to-I in the DNA model.

### II. Experimental results

### 1. Sanger sequencing results

### 1.1 Detection results of A-to-I conversion efficiency under reaction conditions A, B or C

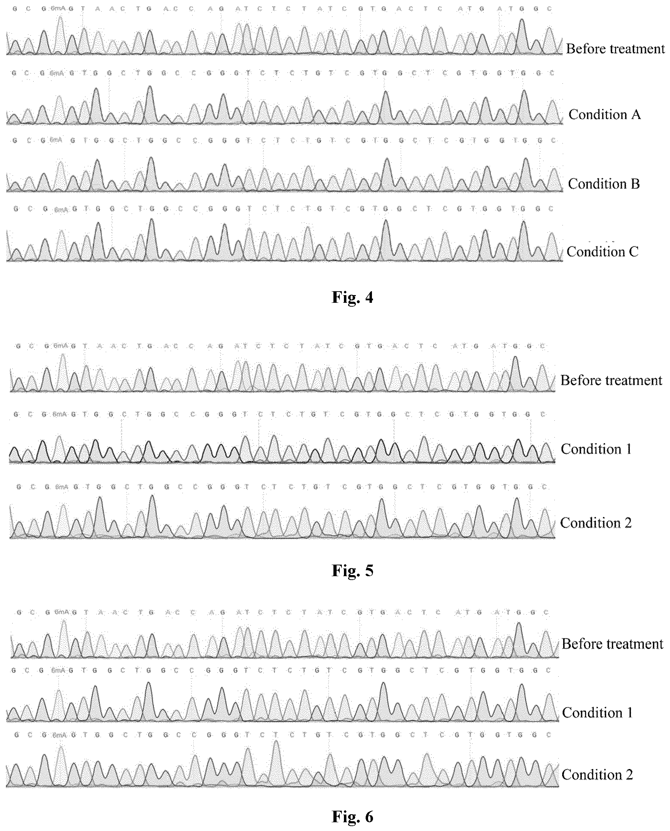

After undergoing the treatment with reaction conditions A, B or C, the exemplary peak map of Sanger sequencing of the amplified product of the DNA model was shown in Fig. 4. As mentioned before, A was converted to I after deamination. I underwent the amplification process to generate a G signal during sequencing and 6mA was still recognized as A after the reaction. Through the Sanger sequencing signal, the A-to-G signal conversion probability in the amplification product as compared to the original sequence (i.e., the sequence before processing) was calculated to obtain the A-to-I conversion rate. The calculation method of the A-to-G signal conversion probability (taking the last A at the 3' end of the sequence as an example) was as follows:
(i) The software 4Peaks was used to calculate the peak height of signal A1 of the remaining A (green signal) and the peak height of signal G1 (gray signal) of the converted G, and then the proportion of unconverted dA was calculated: a=A1/(A1+ G1);
(ii) The efficiency of A-to-I was calculated: b=(1-a)* 100%;
(iii) The conversion efficiencies of all A except 6mA in the mode sequence were calculated, and averaged.

At the same time, the 6mA site information in the original sequence could be determined by detecting the residue of the A signal in the treated DNA model. Though G could also be nonspecifically deaminated (to X (xanthine)), its efficiency was lower than the sequencing baseline, so the nonspecific conversion of G could not be assessed in the Sanger sequencing. It could be seen from the results in Fig. 4 that after being treated with reaction conditions A, B or C, in the amplification product of the treated DNA model, the position of A in the original sequence would always generate G signals, and the signals at the positions of 6mA and C in the original sequence remained unchanged. This result showed that the reaction conditions A, B or C could achieve high specificity and high activity of the A-to-I conversion in the DNA sequences, thereby achieving the detection of a 6mA site in DNA at single-base resolution.

The catalytic reaction conditions for nitrosation reaction corresponding to the reaction conditions A, B, or C and the sequencing signal conversion estimation results of the amplified products were shown in Table 2, in which all concentrations were final concentrations. The final concentration of glyoxal in the nitrosation reaction was the sum of that obtained by volume conversion of the glyoxal added during the nucleic acid protection process and that of the exogenously added glyoxal during the nitrosation reaction.

**Table 2**

| Reaction condition | Nitrosation reagent | Buffer pH6.0 | Glyoxal concentration for protection | Amount of exogenously added glyoxal | Final concentration of glyoxal in the nitrosationm reaction system | Incubation with boric acid (50M) | Time (h) | Temperature (°C) | A-to-G conversion rate |
|---|---|---|---|---|---|---|---|---|---|
| A | 750 mM NaNO₂ | 40 mM MES | 1.3 M | 0 | 0.52 M | Mode 1 | 24 | 16 | 93% |
| B | 750 mM NaNO₂ | 40 mM MES | 1.3 M | 0.88 M | 1.4 M | Mode 1 | 8 | 16 | 96% |
| C | 750 mM NaNO₂ | 40 mM MES | 1.3 M | 0.88 M | 1.4 M | Mode 2 | 8 | 16 | 98% |

| | | | | | | | | | |
|---|---|---|---|---|---|---|---|---|---|
| Note: For the incubation with boric acid, "Mode 1" referred to adding boric acid to the nitrosation reaction system, and "Mode 2" referred to adding boric acid to the nucleic acid protection system after the protection of the nucleic acid with glyoxal and before the nitrosation reaction. | | | | | | | | | |

### 1.2 Effects of different kinds of nitrosating reagents on the A-to-I conversion rate

The effects of different kinds of nitrosating reagents on the conversion rate of A was evaluated. In short, NaNO₂ in the nitrosation reaction system with reaction Condition A in Step 1.2 of the above experimental method was replaced with KNO₂. The specific catalytic reaction conditions and the estimated results of the sequencing signal conversion of the amplified products were shown in Table 3, and the rest steps or reaction conditions not mentioned herein were the same as Condition A.

**Table 3**

| Reaction condition | Nitrosation reagent | Buffer | Glyoxal concentration for protection | Tim e (h) | Temperatur e (°C) | A-to-G conversion rate |
|---|---|---|---|---|---|---|
| 1 | 1.0M KNO₂ | 40mM MES (pH 5.0) | 1.3 M | 24 | 16 | About 95% |
| 2 | 1.5M NaNO₂ | 40mM MES (pH 6.0) | 1.3 M | 24 | 16 | About 93% |

The Sanger sequencing results of the amplified products under various reaction conditions were shown in Fig. 5. From Table 3 and Fig. 5, it could be seen that both NaNO₂ and KNOz could effectively catalyze the conversion of A-to-I.

### 1.3 Effect of NaNO₂ concentration on the A-to-I conversion rate

In this experiment, the effect of different NaNO₂ concentrations in the nitrosation reaction on the A-to-I conversion rate was studied. In short, by adjusting the NaNO₂ concentration in the nitrosation reaction system in Step 1.2 of the above experimental method, the effect of NaNO₂ concentration on the A-to-I conversion rate was studied. The specific catalytic reaction conditions and sequencing signal conversion estimation results of the amplified products were shown in Table 4. The rest steps or reaction conditions not mentioned herein were the same as Condition B.

**Table 4**

| Reaction condition | NaNO₂ | Glyoxal concentration for protection | Amount of exogenously added glyoxal | Final concentration of glyoxal for the nitrosation reaction | Time (h) | Temperatur e (°C) | A-to-G conversion rate |
|---|---|---|---|---|---|---|---|
| 1 | 0.75 M | 1.3 M | 220 mM | 0.75 M | 8 | 16 | About 93% |
| 2 | 1.25 M | 1.3 M | 220 mM | 0.75 M | 18 | 16 | About 95% |

The Sanger sequencing results of the amplified products under each reaction condition were shown in Fig. 6. It could be seen from Table 4 and Fig. 6 that within the NaNO₂ concentration range as shown in Table 4, the conversion rates of A were close to complete (e.g., not less than 93%).

### 1.4 Effect of KNO₂ concentration on the A-to-I conversion rate

In this experiment, the effect of different KNOz concentrations in the nitrosation reaction on the A-to-I conversion rate was studied. In short, by adjusting the KNO₂ concentration in the nitrosation reaction system in Step 1.2 of the above experimental method, the effect of KNO₂ concentration on the A-to-I conversion rate was studied. The specific catalytic reaction conditions and sequencing signal conversion estimation results of the amplified products were shown in Table 5. The rest steps or reaction conditions not mentioned herein were the same as Condition A.

**Table 5**

| Reaction condition | KNO₂ | Glyoxal concentration for protection | Buffer(pH5.5) | Time (h) | Temperature (°C) | A-to-G conversion rate |
|---|---|---|---|---|---|---|
| 1 | 1.5 M | 1.3 M | 40 mM MES | 24 | 16 | About 93% |
| 2 | 1.25 M | 1.3 M | 40 mM MES | 24 | 16 | About 93% |
| 3 | 1.0 M | 1.3 M | 40 mM MES | 24 | 16 | About 95% |
| 4 | 0.75 M | 1.3 M | 40 mM MES | 24 | 16 | About 94% |

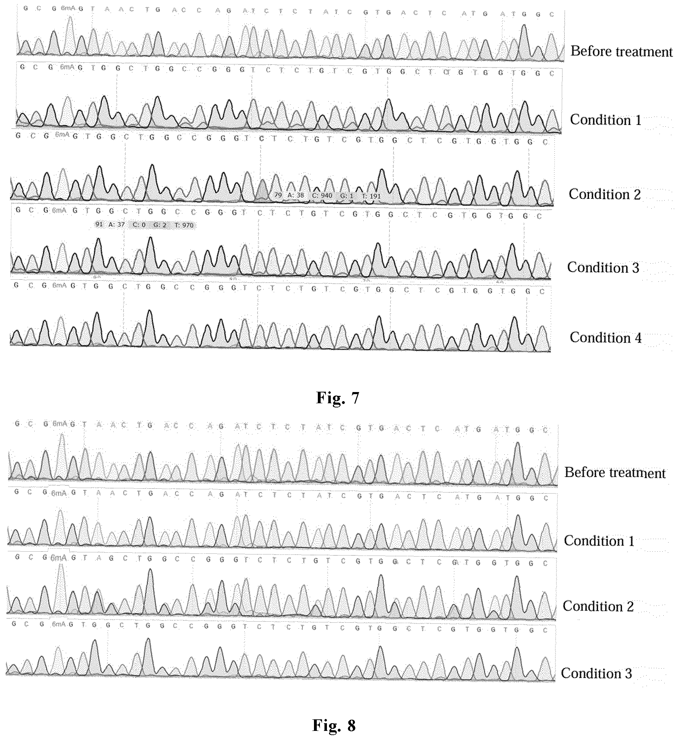

The Sanger sequencing results of the amplified products under various reaction conditions were shown in Fig. 7. It could be seen from Table 5 and Fig. 7 that within the KNO₂ concentration range as shown in Table 5, the conversion rates of A were close to complete (e.g., not less than 93 %).

### 1.5 Effect of the final concentration of glyoxal in nitrosation reaction on the A-to-I conversion rate

In this experiment, the effect of the final concentration of glyoxal in the nitrosation reaction on the A-to-I conversion rate was studied. Briefly, by adjusting the final concentration of glyoxal in Step 1.2 Nitrosation reaction system of the above experimental method, the A-to-I conversion rate was estimated. The specific catalytic reaction conditions and sequencing signal conversion estimation results of the amplified products were shown in Table 6. The rest steps or reaction conditions not mentioned herein were the same as Condition A.

**Table 6**

| Reaction condition | Final concentration of glyoxal in the nitrosation reaction | Time (h) | Temperature (°C) | A-to-G conversion rate |
|---|---|---|---|---|
| 1 | 0 | 18 | 16 | About 0% |
| 2 | 105 mM | 18 | 16 | About 50% |
| 3 | 1.4 M | 8 | 16 | About 95% |

The Sanger sequencing results of the amplified products under various reaction conditions were shown in Fig. 8. It could be seen from Table 6 and Fig. 8 that increasing the amount of exogenously added glyoxal during the nitrosation reaction could increase the A-to-I conversion rate, that was, the higher the final concentration of glyoxal in the nitrosation reaction, the higher the A-to-I conversion rate, and the shorter the reaction time.

### 1.6 Effect of glyoxal concentration during nucleic acid protection on the A-to-I conversion rate

In this experiment, the effect of glyoxal concentration during nucleic acid protection on the A-to-I conversion rate was studied. Briefly, by adjusting the concentration of glyoxal in Step 1.1 Nucleic acid protection of the above experimental method, the effect of glyoxal concentration on the A-to-I conversion rate was studied. The specific catalytic reaction conditions and sequencing signal conversion estimation results of the amplified products were shown in Table 7. The rest steps or reaction conditions not mentioned herein were the same as Condition B.

**Table 7**

| Reaction conditio n | Glyoxal concentration (nucleic acid protection) | Amount of exogenously added glyoxal | Final concentration of glyoxal in the nitrosation reaction | Time (h) | Temperatur e (°C) | A-to-G conversion rate |
|---|---|---|---|---|---|---|
| 1 | 0.32 M | 0.88 M | 1.0 M | 8 | 16 | About 97% |
| 2 | 0.08 M | 0.88 M | 0.91 M | 8 | 16 | About 89% |
| 3 | 0.02 M | 0.88 M | 0.89 M | 8 | 16 | About 87% |
| 4 | 2.6 M | 0.88 M | 1.92 M | 8 | 16 | About 98% |

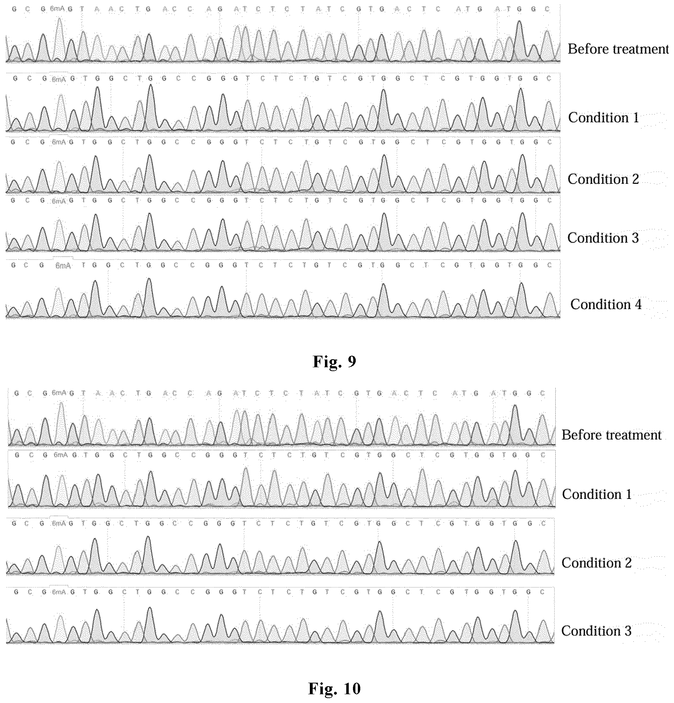

The Sanger sequencing results of the amplified products under each reaction condition were shown in Fig. 9. It could be seen from Table 7 and Fig. 9 that under reaction conditions 1 to 4, the conversion rates of A were all 87% or above, and, the higher the glyoxal concentration in the nucleic acid protection process, the higher the conversion rate of A.

### 1.7 Effect of amount of exogenously added glyoxal in nitrosation reaction on the A-to-I conversion rate

In this experiment, the effect of the amount of exogenously added glyoxal in the nitrosation reaction on the A-to-I conversion rate was studied. In short, by adjusting the amount of exogenously added glyoxal in Step 1.2 Nitrosation reaction of the above experimental method, the effect of the amount of exogenously added glyoxal on the A-to-I conversion rate was studied. The specific catalytic reaction conditions and sequencing signal conversion estimation results of the amplified products were shown in Table 8. The rest steps or reaction conditions not mentioned herein were the same as Condition B.

**Table 8**

| Reaction conditio n | Glyoxal concentration (nucleic acid protection) | Amount of exogenously added glyoxal | Final concentration of glyoxal in the nitrosation reaction | Tim e (h) | Temperature (°C) | A-to-G conversion rate |
|---|---|---|---|---|---|---|
| 1 | 1.3 M | 0 | 0.52 M | 24 | 16 | About 93% |
| 2 | 1.3 M | 0.88 M | 1.4 M | 8 | 16 | About 96% |
| 3 | 1.3 M | 0.02 M | 0.55 M | 8 | 16 | About 94% |

The Sanger sequencing results of the amplified products under each reaction condition were shown in Fig. 10. It could be seen from Table 8 and Fig. 10 that under reaction conditions 1 to 3, the conversion rates of A were all 93% or above, and during the nitrosation reaction, the greater the amount of exogenously added glyoxal, the higher the conversion rate of A. In addition, according to the conversion rate results in Tables 7 to 8, it was further confirmed that the higher the final concentration of glyoxal in the nitrosation reaction system, the higher the A-to-I conversion rate.

### 1.8 Effect of incubation conditions during nucleic acid protection process on the A-to-I conversion rate

In this experiment, the effect of nucleic acid protection incubation conditions on the A-to-I conversion rate was studied. Briefly, by adjusting the temperature and time of the incubation of glyoxal and nucleic acid in Step 1.1 Nucleic acid protection of the above experimental method, the effects of the temperature and time on the A-to-I conversion rate were studied. The specific nucleic acid protection and catalytic reaction conditions, as well as the sequencing signal conversion estimation results of the amplified products were shown in Table 9. The rest steps or reaction conditions not mentioned herein were the same as Condition A.

**Table 9**

| Reaction condition | Glyoxal concentration (nucleic acid protection) | Incubation conditions for nucleic acid protection | Time (h) | Temperature (°C) | A-to-G conversion rate |
|---|---|---|---|---|---|
| 1 | 1.3 M | 50°C 30 min | 8 | 16 | About 89% |
| 2 | 1.3 M | 50°C 15 min | 8 | 16 | About 90% |
| 3 | 1.3 M | 37°C 60 min | 8 | 16 | About 83% |
| 4 | 1.3 M | 37°C 30 min | 8 | 16 | About 85% |
| 5 | 1.3 M | 25°C 60 min | 8 | 16 | About 84% |
| 6 | 1.3 M | 16°C 60 min | 8 | 16 | About 85% |

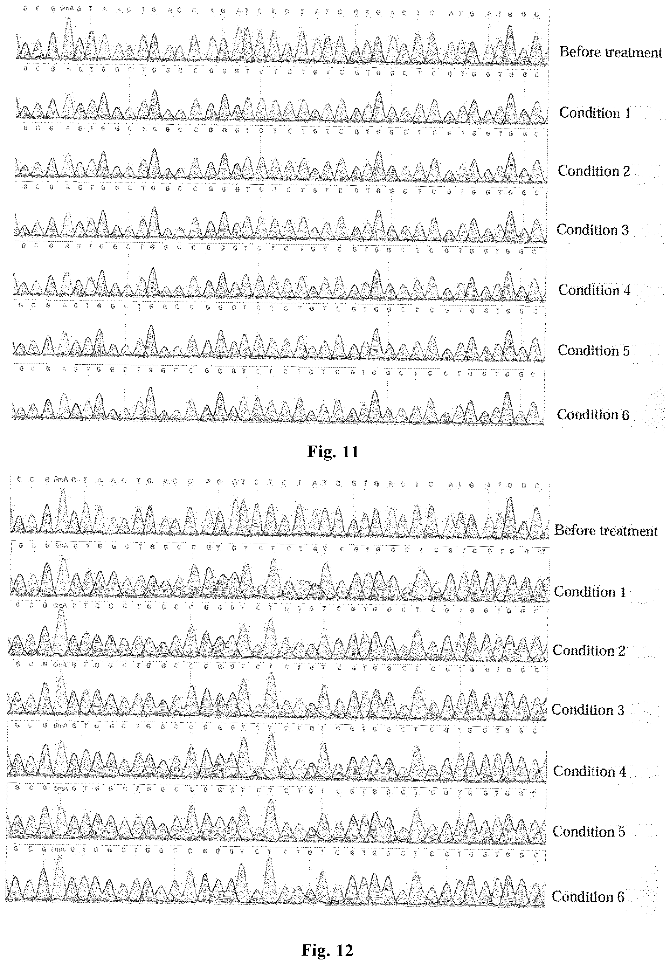

The Sanger sequencing results of the amplified products under each reaction condition were shown in Fig. 11. From Table 9 and Fig. 11, it could be seen that when the incubation temperature of glyoxal and nucleic acid during the nucleic acid protection process was in the range of 16 to 50°C, and the incubation time was in the range of 15 to 60 minutes, they would not affect the conversion rate of A.

### 1.9 Effect of buffer solution and boric acid concentration on the A-to-I conversion rate

In this experiment, the effects of buffer solution and boric acid concentration on the A-to-I conversion rate were studied. In short, by adjusting the type of buffer solution and boric acid concentration in Step 1.2 Nitrosation reaction step of the above experimental method, the effects of the type of buffer solution and boric acid concentration on the A-to-I conversion rate were studied. The specific nucleic acid protection and catalytic reaction conditions, as well as the sequencing signal conversion estimation results of the amplified products were shown in Table 10 (glyoxal was not exogenously added during nitrosation reaction) and Table 11 (glyoxal was exogenously added during nitrosation reaction), and the rest steps or reaction conditions not mentioned herein were the same as the condition A.

**Table 10**

| Reaction condition | NaNO₂ | Buffer | H₃BO₃ | Time (h) | Temperature (°C) | A-to-G conversion rate |
|---|---|---|---|---|---|---|
| 1 | 1.0 M | 40 mM MES (pH 5.0) | 5 mM | 18 | 22 | About 95% |
| 2 | 1.0 M | 40 mM MES (pH 5.0) | 20 mM | 18 | 22 | About 89% |
| 3 | 1.25 M | 40 mM MES (pH 5.0) | 50 mM | 18 | 16 | About 93% |
| 4 | 1.0 M | 300 mM NaOAc (pH 5.2) | 5 mM | 18 | 22 | About 92% |
| 5 | 1.0 M | 300 mM NaOAc (pH 5.2) | 20 mM | 18 | 22 | About 90% |
| 6 | 1.0 M | 300 mM NaOAc (pH 5.2) | 50 mM | 18 | 22 | About 95% |

**Table 11**

| Reaction conditio n | Buffer | H₃BO₃ | Glyoxal concentration (nucleic acid protection) | Amount of exogenously added glyoxal | Time (h) | Temperature (°C) | A-to-G conversio n rate |
|---|---|---|---|---|---|---|---|
| 1 | 100 mM MES (pH 5.5) | 50 mM | 1.3 M | 0.88 M | 8 | 16 | About 98% |
| 2 | 100 mM MES (pH 5.5) | 75 mM | 1.3 M | 0.88 M | 8 | 16 | About 98% |
| 3 | 100 mM MES (pH 5.5) | 100 mM | 1.3 M | 0.88 M | 8 | 16 | About 98% |
| 4 | H₂O | 50 mM | 1.3 M | 0.88 M | 8 | 16 | About 94% |

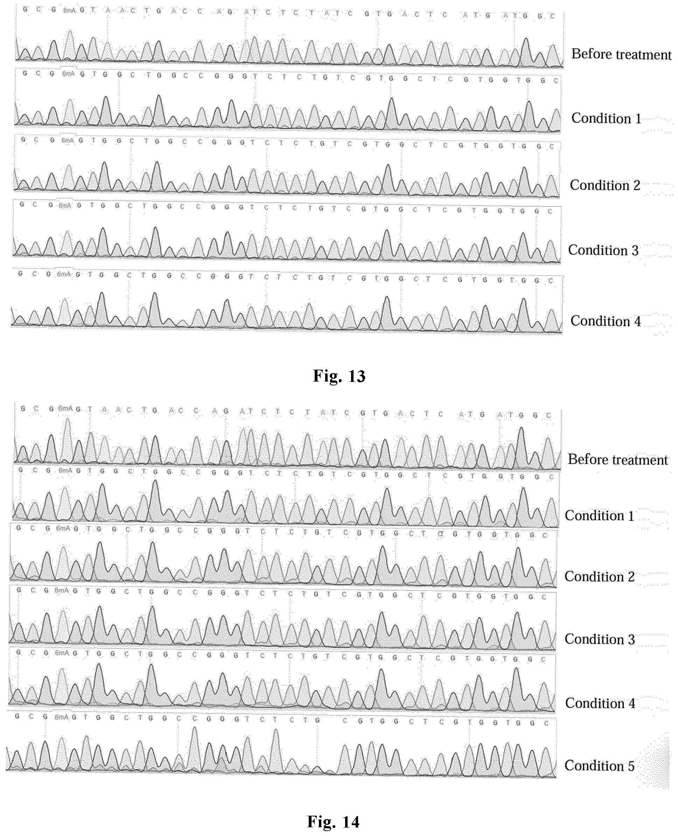

The Sanger sequencing results of the amplified products under each reaction condition in Tables 10 and 11 were shown in Figs. 12 and 13, respectively. The results showed that the boric acid concentration at 5 to 100 mM in the nitrosation reaction system did not affect the conversion of A-to-I; in addition, the results in Table 10 and Table 11 also showed that when MES was replaced with NaOAc, or water was directly used as the solvent, the conversion efficiency of A-to-I was almost unaffected.

### 1.10 Effect of MES buffer solution concentration and pH value on the A-to-I conversion rate

In this experiment, the effects of the MES buffer solution concentration and pH value in the nitrosation reaction on the A-to-I conversion rate were studied. Briefly, by adjusting the concentration and pH value of the MES buffer solution in Step 1.2 Nitrosation reaction of the above experimental method, the effects of the concentration and pH value of the MES buffer solution on the A-to-I conversion rate were studied. The specific nucleic acid protection and catalytic reaction conditions, as well as the sequencing signal conversion estimation results of the amplified products were shown in Table 12 (different MES concentrations) and Table 13 (different pH values). The rest steps or reaction conditions not mentioned here were the same as Condition A.

**Table 12**

| Reaction condition | BufferMES | H₃BO₃ | Glyoxal concentration (nucleic acid protection) | Time (h) | Temperature (°C) | A-to-G conversion rate |
|---|---|---|---|---|---|---|
| 1 | 20 mM pH5.0 | 10 mM | 1.3 M | 18 | 22 | About 90% |
| 2 | 40 mM pH5.0 | 10 mM | 1.3 M | 12 | 22 | About 95% |
| 3 | 80 mM pH5.0 | 40 mM | 1.3 M | 18 | 22 | About 93% |
| 4 | 100 mM pH5.0 | 50 mM | 1.3 M | 12 | 22 | About 91% |
| 5 | 750 mM pH5.0 | 50 mM | 1.3 M | 8 | 16 | About 96% |

**Table 13**

| Reaction condition | BufferMES | H₃BO₃ | Glyoxal concentration (nucleic acid protection) | Amount of exogenously added glyoxal | Time (h) | Temperature (°C) | A-to-G conversion rate |
|---|---|---|---|---|---|---|---|
| 1 | 100 mM pH 5.5 | 50 mM | 1.3 M | 0.88 M | 8 | 16 | About 96% |
| 2 | 100 mM pH 4.0 | 50 mM | 1.3 M | 0.88 M | 8 | 16 | About 96% |
| 3 | 250 mM pH 5.0 | 50 mM | 1.3 M | 0.88 M | 8 | 16 | About 92% |
| 4 | 250 mM pH 4.5 | 50 mM | 1.3 M | 0.88 M | 8 | 16 | About 93% |
| 5 | 250 mM pH 4.0 | 50 mM | 1.3 M | 0.88 M | 8 | 16 | About 91% |

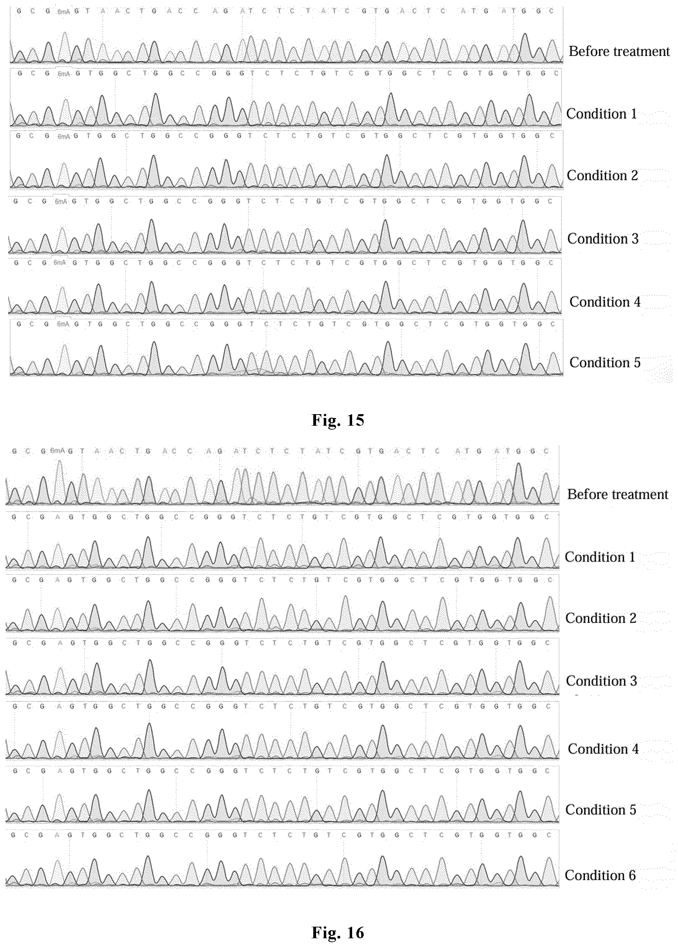

The Sanger sequencing results of the amplified products under each reaction condition in Tables 12 and 13 were shown in Figs. 14 and 15, respectively. The results showed that the MES buffer solutions with different concentrations (e.g., 20 to 750 mM) and different pH values (e.g., 4.0 to 5.5) all could effectively promote the A-to-I conversion.

### 1.11 Effect of boric acid or salt thereof on the A-to-I conversion rate

In this experiment, the effect of boric acid or salt thereof in the nitrosation reaction on the A-to-I conversion rate was studied. Briefly, the boric acid in Step 1.2 Nitrosation reaction of the above experimental method was replaced with potassium borate with different pH values to estimate the A-to-I conversion rate. The specific nucleic acid protection and catalytic reaction conditions, as well as the sequencing signal conversion estimation results of the amplification products were shown in Table 14. The rest steps or reaction conditions not mentioned herein were the same as Condition B.

**Table 14**

| Reaction condition | Boric acid or salt thereof 50 mM | Glyoxal concentration (nucleic acid protection) | Amount of exogenously added glyoxal | Time (h) | Temperatur e (°C) | A-to-G conversion rate |
|---|---|---|---|---|---|---|
| 1 | K₃BO₃ pH8.8 | 1.3 M | 0.44 M | 8 | 16 | About 93% |
| 2 | K₃BO₃ pH8.7 | 1.3 M | 0.44 M | 8 | 16 | About 93% |
| 3 | K₃BO₃ pH8.6 | 1.3 M | 0.44 M | 8 | 16 | About 94% |
| 4 | K₃BO₃ pH8.5 | 1.3 M | 0.44 M | 8 | 16 | About 96% |
| 5 | K₃BO₃ pH8.4 | 1.3 M | 0.44 M | 8 | 16 | About 93% |
| 6 | K₃BO₃ pH8.3 | 1.3 M | 0.44 M | 8 | 16 | About 92% |

The Sanger sequencing results of the amplified products under various reaction conditions were shown in Fig. 16. From Table 14 and Fig. 16, it could be seen that after replacing boric acid in the nitrosation reaction with borate, the A-to-I conversion rate was not affected (e.g., all were 92% or above).

### 1.12 Effect of boric acid incubation mode on the A-to-I conversion rate

In this experiment, the effect of boric acid incubation mode on the A-to-I conversion rate was studied. Briefly, by adjusting the boric acid incubation mode in the above experimental method, the A-to-I conversion rate was estimated. The specific nucleic acid protection and catalytic reaction conditions, as well as the sequencing signal conversion estimation results of the amplification products were shown in Table 15. The rest steps or reaction conditions not mentioned herein were the same as Condition B. Among them, "Mode 1" of boric acid incubation referred to adding boric acid to the nitrosation reaction system; "Mode 2" referred to adding boric acid to the nucleic acid protection system after the nucleic acid was protected by glyoxal and before the nitrosation reaction; "Mode 3" referred to incubating glyoxal and boric acid, then using the incubation product of glyoxal and boric acid to protect nucleic acid.

**Table 15**

| Reaction condition | MES buffer solution | H₃BO₃ | Boric acid incubation mode | Glyoxal concentration (nucleic acid protection) | Amount of exogenously added glyoxal | A-to-G conversion rate |
|---|---|---|---|---|---|---|
| 1 | 40 mM pH 6.0 | 50 mM | Mode 1 | 1.3 M | 0.88 M | About 97% |
| 2 | 40 mM pH 6.0 | 50 mM | Mode 3 | 0.88 M | 0.88 M | About 96% |
| 3 | 40 mM pH 6.0 | 50 mM | Mode 3 | 0.32 M | 0.88 M | About 96% |
| 4 | 40 mM pH 6.0 | 50 mM | Mode 2 | 1.3 M | 0.88 M | About 98% |

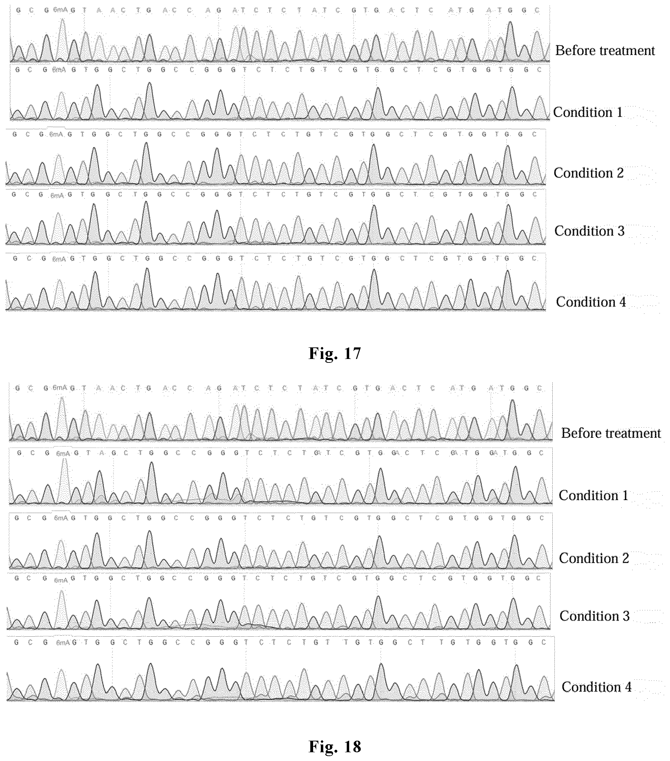

The Sanger sequencing results of the amplified products under each reaction condition were shown in Fig. 17. From Table 15 and Fig. 17, it could be seen that under the above three boric acid incubation modes, the conversion rates of A were all 96% or above, and, when the incubation with H₃BO₃ was carried out by Mode 2, the conversion rate of A was relatively higher, but showed no significant difference.

### 1.13 Effect of nitrosation reaction temperature on the A-to-I conversion rate

In this experiment, the effect of nitrosation reaction temperature on the A-to-I conversion rate was studied. In short, by adjusting the reaction temperature and time in Step 1.2 Nitrosation reaction of the above experimental method, the effect of the nitrosation reaction temperature on the A-to-I conversion rate was studied. The specific nucleic acid protection and catalytic reaction conditions, as well as the sequencing signal conversion estimation results of the amplified products were shown in Table 16. The rest steps or reaction conditions not mentioned herein were the same as Condition B.

**Table 16**

| Reaction condition | NaNO₂ | Glyoxal concentration (nucleic acid protection) | Amount of exogenously added glyoxal | Time (h) | Temperatur e (°C) | A-to-G conversion rate |
|---|---|---|---|---|---|---|
| 1 | 0.75 M | 1.3 M | 0.88 M | 1 | 16 | About 69% |
| 2 | 0.75 M | 1.3 M | 0.88 M | 8 | 16 | About 92% |
| 3 | 0.75 M | 1.3 M | 0.88 M | 0.5 | 50 | About 90% |
| 4 | 0.75 M | 1.3 M | 0.88 M | 1 | 50 | About 92% |

The Sanger sequencing results of the amplified products under each reaction condition were shown in Fig. 18. From Table 16 and Fig. 18, it could be seen that the nitrosation reaction could occur in the temperature range of 16 to 50°C, and, the higher the temperature, the shorter the reaction time required.

### 1.14 Effect of carbonyl compound type on the A-to-I conversion rate

In this experiment, the effect of the types of carbonyl compounds on the A-to-I conversion rate was studied. In short, the glyoxal used in Step 1.1 nucleic acid protection and Step 1.2 nitrosation reaction in the above experimental method was replaced with carbonyl compounds of different types and concentrations, the effect of the types and concentrations of carbonyl compounds on the A-to-I conversion rate was studied. The specific nucleic acid protection and catalytic reaction conditions, as well as the sequencing signal conversion estimation results of the amplified products were shown in Table 17. The rest steps or reaction conditions not mentioned herein were the same as Condition B.

**Table 17**

| Reaction condition | Nucleic acid protection | Exogenous addition for nitrosation reaction | Time (h) | Temperature (°C) | A-to-G conversion rate |
|---|---|---|---|---|---|
| 1 | 1.3 M glyoxal | 105 mM glyoxal | 18 | 16 | About 92% |
| 2 | 1.3 M glyoxal | 67 mM pyruvaldehyde | 18 | 16 | About 93% |
| 3 | 1.3 M glyoxal | 136 mM 2,3-butanedione | 18 | 16 | About 92% |
| 4 | 1.3 M glyoxal | / | 24 | 16 | About 91% |
| 5 | 1.3 M glyoxal | 400 mM phenylglyoxal | 8 | 16 | About 90% |
| 6 | 1.3 M glyoxal | 400 mM 2-bromomalondialdehyde | 8 | 16 | About 95% |
| 7 | 1.3 M glyoxal | 400 mM trichloroacetaldehyde | 8 | 16 | About 91% |
| 8 | 1.3 M glyoxal | 880 mM glyoxal | 8 | 16 | About 88% |
| 9 | 0.15 M ninhydrin | 880 mM glyoxal | 8 | 16 | About 92% |
| 10 | 1.3 M glyoxal | 100 mM ninhydrin | 8 | 16 | About 91% |
| 11 | 1.3 M glyoxal | / | 24 | 16 | About 90% |

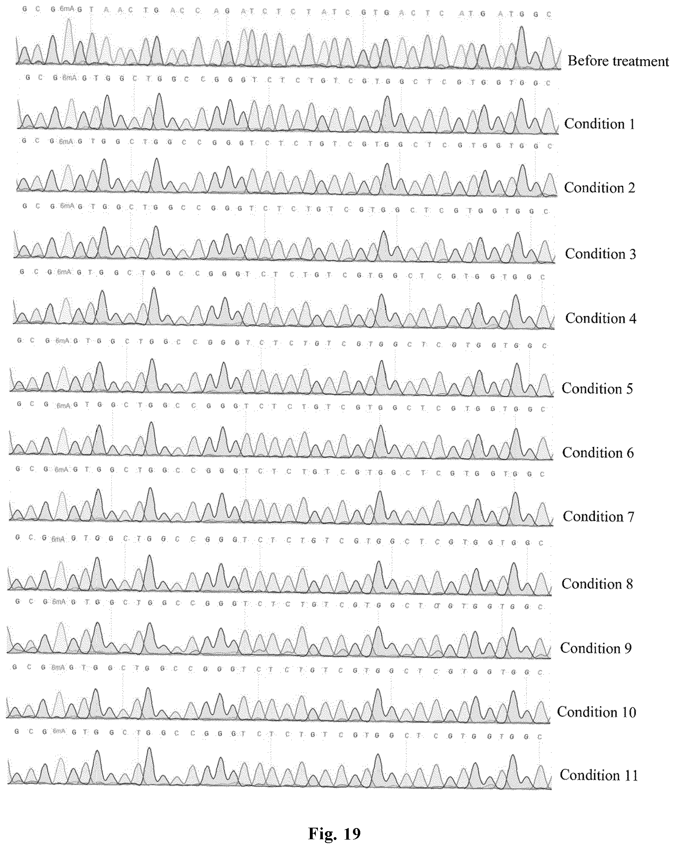

The Sanger sequencing results of the amplified products under various reaction conditions were shown in Fig. 19. From Table 17 and Fig. 19, it could be seen that after using high-concentration glyoxal (e.g., 1.3 M glyoxal) for nucleic acid protection, in the nitrosation reaction system, adding different carbonyl compounds (e.g., glyoxal, pyruvaldehyde, 2,3-butanedione, phenylglyoxal, 2-bromomalondialdehyde, trichloroacetaldehyde, ninhydrin, etc.) could promote the A-to-I conversion, and the conversion rates could all reached 88% or above; in addition, ninhydrin, as a carbonyl compound, could not only catalyze the nitrosation reaction, but also protect nucleic acids, and its effect was similar to that of glyoxal.

### 2. Triple tandem quadrupole mass spectrometry (QQQ) detection results

As mentioned before, the Sanger sequencing method comprised that the conversion rate of A was detected by the signal conversion of A-to-G in the DNA amplification product after the nitrosation reaction, wherein the signal conversion of A-to-G was caused by the A-to-I conversion and the pairing of I and C occurs during the DNA amplification. Therefore, the accuracy of complementary base pairing during the amplification process could affect the measurement results of the conversion rate. Thus, we further used mass spectrometry to directly measure the I content in the nitrosation reaction product, and calculated the conversion rate of A by calculating the ratio of I/(A+I) in the product, and the conversion rate calculated from the Sanger sequencing results was further verified.

The QQQ detection results of DNA samples treated with different NaNO₂ concentrations, different buffer solution types and concentrations, as well as different H₃BO₃ concentrations and incubation modes were shown in Table 18 and Table 19. The rest steps or reaction conditions not mentioned herein were the same as Condition B.

**Table 18**

| Reaction condition | Nitrite | Buffer | Boric acid or salt thereof 50 mM | Time (h) | Amount of exogenously added glyoxal | A-to-I conversion rate |
|---|---|---|---|---|---|---|
| 1 | 1.0 M KNO₂ | 40mM MES (pH5.0) | H₃BO₃ | 24 | / | 100% |
| 2 | 0.75 M KNO₂ | 100mM MES (pH5.5) | H₃BO₃ | 24 | / | 100% |
| 3 | 0.75 M NaNO₂ | 40mM MES (pH6.0) | H₃BO₃ | 24 | / | 100% |
| 4 | 0.75 M NaNO₂ | 40mM MES (pH6.0) | H₃BO₃ | 8 | 0.88 M | 100% |
| 5 | 1.0 M NaNO₂ | 40mM MES (pH5.0) | H₃BO₃ | 24 | / | 100% |
| 6 | 1.0 M NaNO₂ | 300mM NaOAc (pH5.0) | H₃BO₃ | 24 | / | 100% |
| 7 | 0.75 M NaNO₂ | 40mM MES (pH6.0) | K₃BO₃ | 24 | / | 100% |
| 8 | 0.75 M NaNO₂ | H₂O | H₃BO₃ | 8 | 0.88 M | 100% |

**Table 19**

| Reaction condition | Buffer | H₃BO₃ | Boric acid incubation mode | Glyoxal concentration (nucleic acid protection) | Amount of exogenously added glyoxal | A-to-I conversion rate |
|---|---|---|---|---|---|---|
| 1 | 40mM MES (pH6.0) | 50 mM | Mode 2 | 1.3 M | 0.88 M | 100% |
| 2 | 40mM MES (pH6.0) | 50 mM | Mode 2 | 1.3 M | 0.22 M | 100% |
| 3 | 100mM MES (pH5.5) | 50 mM | Mode 2 | 1.3 M | 0.88 M | 100% |
| 4 | 100mM MES (pH5.5) | 100 mM | Mode 2 | 1.3 M | 0.88 M | 100% |

| | | | | | | |
|---|---|---|---|---|---|---|
| Note: Boric acid incubation, "Mode 2" referred to adding boric acid to the nucleic acid protection system after the nucleic acid was protected by glyoxal and before the nitrosation reaction. | | | | | | |

The results in Tables 18 and 19 showed that the conversion of A-to-I in DNA samples tended to be complete under the above conditions. Therefore, the QQQ detection results further verified that the method of the present application could achieve efficient conversion of A-to-I, thereby enabling accurate detection of 6mA modification levels and 6mA modification sites in nucleic acid samples.

### Example 2: Detection of 6mA in genomic DNA

This example further verified the effect of the method of the present application on detecting 6mA at the genome level.

### I. Experimental methods

### 1. Extraction and fragmentation of genomic DNA

A bacterial genomic DNA extraction kit (purchased from Tiangen, Cat. No.: DP302) was used to extract the genomic DNA of *E. coli* k12 mg1655 strain (purchased from ATCC (American Type Culture Collection), Cat. No.: 700926D-5), a non-contact ultrasonic fragmentation instrument (Covaris M220) was used to fragment the extracted DNA into approximately 550 bp fragments, then the DNA samples were spin-dried and placed on ice for later use.

### 2. Denaturation of nucleic acid

### 2.1 Denaturation with formamide

1µg of the fragmented DNA obtained in step 1 was taken and dissolved into 2.8 µL of 85% deionized formamide aqueous solution at 95°C for 10 minutes, then immediately placed on ice for 2 minutes and immediately used in the next step for nucleic acid protection.

### 2.2 Denaturation with 0.1M NaOH

1µg of the fragmented DNA obtained in step 1 was taken and dissolved into 2.0 µL of 0.1 M NaOH aqueous solution at 95°C for 10 minutes, immediately placed on ice for 2 minutes, then added with 0.8 µL of 500 mM MES solution, adjusted to have a pH of 6, placed on ice, and immediately used in the next step for nucleic acid protection.

Note: For nucleic acid denaturation, either 2.1 Denaturation with formamide or 2.2 Denaturation with NaOH could be chosen.

### 3. Conversion reaction of A (adenine) to I (hypoxanthine)

The reaction condition C same as Example 1 was used.

### 4. Purification of conversion product

The step 2 in Example 1 was used.

### 5. Triple tandem quadrupole mass spectrometry (QQQ) detection

5.1 A mass of 500 ng of the prepared DNA was taken, added with 1U of nuclease P1, 2 µL of NH₄OAc (pH=5.3), replenished with water to 20 µL, and reacted at 42°C for 2 hours; then added with 1 µL of FastAP Thermosensitive Alkaline Phosphatase, 2.5 µL of 10× FastAP buffer, replenished with water to 25 µL, and reacted at 37°C for 2 hours.

The above reaction solution was centrifuged at 4°C and 12000 rpm for 10 minutes, and 25 µL thereof was taken for QQQ detection to measure the I content in the sample DNA after the nitrosation reaction, thereby further determining the conversion efficiency of A-to-I in the DNA.

### II. Experimental results

After the formamide denaturation, the alkali denaturation or no denaturation pretreatment, the QQQ detection results of each genomic DNA reaction sample were shown in Table 20.

**Table 20**

| Reaction condition | Denaturation condition | Glyoxal concentration (nucleic acid protection) | Amount of exogenously added glyoxal | Final concentration of glyoxal in the nitrosation reaction | A-to-I conversion rate |
|---|---|---|---|---|---|
| 1 | Denaturation with NaOH | 1.3 M | 0.88 M | 1.4 M | 100% |
| 2 | Denaturation with formamide | 1.3 M | 0.88 M | 1.4 M | 100% |
| 3 | No denaturation | 1.3 M | 0.88 M | 1.4 M | 100% |

It could be seen from Table 20 that the efficient conversion of A to I could be achieved by the method of the present application under the condition C. Moreover, A in double-stranded DNA without denaturation pretreatment could also be completely converted, indicating that the method of the present application could achieve the conversion of A-to-I in DNA samples, and the conversion efficiency was very high.

### Example 3: Detection of m⁶A in RNA

### I. Experimental methods

### 1. Extraction and purification of RNA

1.1 An RNA extraction kit (Trizol method) (purchased from Invitrogen, Cat. No.: 15596026) was used to extract total RNA from HEK293T cells (purchased from ATCC (American Type Culture Collection), Cat. No. CRL-3216).

1.2 A mass of 1 µg of the total RNA prepared in step 1.1 was taken, diluted with nuclease-free water or DEPC water to 18 µL, added with 2 µL of NEB Next Magnesium RNA Fragmentation ModµLe, incubated at 94°C for 4 minutes, and then cooled to 4°C; subsequently added with 2 µL of stop solution and mixed well, added with 2 µL of Glycogen and mixed well, added with 50 µL of ethanol (final concentration was 70% EtOH) and allowed to stand at -20°C for 1 h; then centrifuged at 12,000 g for 20 minutes, added with 1 ml of 70% ethanol and centrifuged again to remove the liquid.

### 2. Conversion reaction of A (adenine) to I (hypoxanthine)

The conversion reaction of A-to-I could be carried out with reference to the exemplary reaction conditions A, B, C, D or E.

### Exemplary Condition A for conversion reaction

### 2.1 Protection of nucleic acid

The RNA prepared in step 1 was dissolved in 2.8 µL of DEPC water, added with 4 µL of DMSO and 1.2 µL of 40% glyoxal, mixed well, and incubated at 50°C for 1 hour to obtain a nucleic acid protected by glyoxal, which was cooled on ice for later use.

### 2.2 Nitrosation reaction

Preparation of RNA reaction solution: 3 µL of 5 M NaNO₂, 1.6 µL of 500 mM MES (pH 6), 2 µL of H₃BO₃, and 5.4 µL of DEPC water were added with 8 µL of the above nucleic acid protected by glyoxal to reach a total volume of 20 µL, and reacted at 16°C for 8 h.

### Exemplary Condition B for conversion reaction

### 2.1 Protection of nucleic acid

The RNA prepared in step 1 was dissolved in 2.8 µL of DEPC water, added with 4 µL of DMSO and 1.2 µL of 40% glyoxal, mixed, and incubated at 50°C for 1 hour to obtain a nucleic acid protected by glyoxal, which was cooled on ice for later use.

### 2.2 Nitrosation reaction

Preparation of RNA reaction solution: 3 µL of 5 M NaNO₂, 1.6 µL of 500 mM MES (pH 6), 2 µL of H₃BO₃, 3.4 µL of DEPC water, and 2 µL of 40% glyoxal aqueous solution were added with 8 µL of the above nucleic acid protected by glyoxal to reach a total volume of 20 µL, and reacted at 16°C for 8 h.

### Exemplary Condition C for conversion reaction

### 2.1 Protection of nucleic acid

The RNA prepared in step 1 was dissolved in 2.8 µL of DEPC water, added with 4 µL of DMSO and 1.2 µL of 40% glyoxal, mixed well at 50°C for 0.5 h; subsequently, the mixture solution was added with 2 µL of H₃BO₃, and underwent incubation at 50°C for 0.5 hours to obtain a nucleic acid protected by glyoxal, which was cooled on ice for later use.

### 2.2 Nitrosation reaction

Preparation of RNA reaction solution: 3 µL of 5 M NaNO₂, 1.6 µL of 500 mM MES (pH 6), 3.4 µL of DEPC water, and 2 µL of 40% glyoxal aqueous solution were added with 10 µL of the above nucleic acid protected by glyoxal to reach a total volume of 20 µL, and reacted at 16°C for 8 h.

### Exemplary Condition D for conversion reaction

### 2.1 Protection of nucleic acid

The RNA prepared in step 1 was dissolved in 2.8 µL of DEPC water, added with 4 µL of DMSO and 1.2 µL of 40% glyoxal, mixed well at 50°C for 0.5 h; subsequently, the mixture solution was added with 2 µL of H₃BO₃, and underwent incubation at 50°C for 0.5 hours to obtain a nucleic acid protected by glyoxal, which was cooled on ice for later use.

### 2.2 Nitrosation reaction

Preparation of RNA reaction solution: 4 µL of 5 M NaNO₂, 1.6 µL of 500 mM MES (pH 6), 3.4 µL of DEPC water, and 2 µL of 40% glyoxal aqueous solution were added with 10 µL of the above nucleic acid protected by glyoxal to reach a total volume of 20 µL, and reacted at 50°C for 0.5 h.

### Exemplary Condition E for conversion reaction

### 2.1 Protection of nucleic acid

The RNA prepared in step 1 was dissolved in 2.8 µL of DEPC water, added with 4 µL of DMSO and 1.2 µL of 40% glyoxal, mixed well at 50°C for 0.5 h; subsequently, the mixture solution was added with 2 µL of H₃BO₃, and underwent incubation at 50°C for 0.5 hours to obtain a nucleic acid protected by glyoxal, which was cooled on ice for later use.

### 2.2 Nitrosation reaction

Preparation of RNA reaction solution: 3 µL of 5 M NaNO₂ p-toluenesulfonic acid aqueous solution (pH 6), 5 µL of DEPC water, and 2 µL of 40% glyoxal aqueous solution were added with 10 µL of the above nucleic acid protected by glyoxal to reach a total volume of 20 µL, and reacted at 50°C for 0.5 h.

### 3. Purification of conversion product

3.1 The sample prepared in step 2 was added to 2 µL of 3 M NaOAc (pH 5.2), 2 µL of Glycogen, and mixed well, then added with 55 µL of ethanol (the final concentration was 70% EtOH), and incubated at -20°C for 1 h; then centrifuged at 12,000 g for 20 minutes, added with 1 ml of 70% ethanol and centrifuged again to remove the liquid.

3.2 A volume of 50 µL of Deprotection A buffer was added to the above tube, reacted at 95°C for 10 minutes, placed on ice, added with 5 µL of 3 M NaOAc (pH 5.2), 2 µL of Glycogen and mixed well, added with 142 µL of ethanol, incubated at -20°C for 1 hour, then centrifuged at 12,000 g for 20 minutes, added with 1 ml of 70% ethanol and centrifuged again to remove liquid.

3.3 A volume of 50 µL of Deprotection B buffer was added to the above tube, reacted at 65°C for 120 minutes, placed on ice, added with 5 µL of 3 M NaOAc (pH 5.2), 2 µL of Glycogen and mixed well, added with 142 µL of ethanol, incubated at -20°C for 2 hours, then centrifuged at 12,000 g for 10 minutes, added with 1 ml of 70% ethanol and centrifuged again to remove the liquid.

Note: Step 3.3 above was an optional step.

3.4 Using RNA Clean & Concentrator Kits to purify RNA and determining concentration with Nanodrop.

### 4. Construction of cDNA library

Untreated RNA and nitrosated RNA were used for library construction. RNA samples were treated with PNK (polynucleotide kinase, purchased from NEB, Cat. No.: M0201S) to dephosphorylate the 3' end of RNA fragment, the dephosphorylated RNA was ligated to 3' RNA linker (SEQ ID NO: 5) by using T4 RNA ligase2 and truncated KQ (purchased from NEB, Cat. No.: M0373S), and the excessive 3' RNA linker was removed by digestion with 5' Deadenylase (purchased from NEB, Cat. No.: M0331S) and RecJf enzyme (purchased from NEB, Cat. No.: M0264S).

SuperScript III (purchased from Invitrogen, Cat. No.: 18080093) was used to perform reverse transcription of RNA. Silane beads were used to purify the cDNA obtained in reverse transcription. An adapter with 5' phosphorylation (SEQ ID NO: 6) was ligated to the 3' end of the purified cDNA by using T4 RNA ligase 1, high concentration (purchased from NEB, Cat. No.: M0437M). The cDNA to which the 5' adaptor was linked was further purified using silane beads. The cDNA amplification primers 1 and 2 (SEQ ID NOs: 7 to 8, in which "XXXXXX" referred to the index sequence) were used to perform PCR amplification of the cDNA. The PCR product was purified with 8% TBE gel, and the gel fragments with a fragment size between 160 to 250 bp were cut out and the amplification of the cDNA was recovered to obtain a cDNA sequencing library. Finally, the resulting library was sequenced using Illumina Hiseq X10 PE150.

### 5. Triple tandem quadrupole mass spectrometry (QQQ) detection

A mass of 200 ng of the RNA prepared in step 3 was taken, added with 1U of nuclease P1, 2 µL of NH₄OAc, replenished with water to 20 µL, and reacted at 42°C for 2 hours; subsequently, added with 1 µL of FastAP Thermosensitive Alkaline Phosphatase, 2.5 µL of 10× FastAP buffer, replenished with water to 25 µL, and reacted at 37°C for 2 hours.

After the above reaction, the solution was centrifuged at 4°C, 12000 rpm for 10 minutes, and 25 µL thereof was taken for QQQ detection.

### II. Experimental results

### 1. High-throughput sequencing and data analysis

### 1.1 High-throughput data analysis

### 1) Filtering the original data of the present application

After the data was downloaded from the machine, trim_galore (version 0.6.6) software was first used to remove sequencing adapters from the sequencing data (FASTQ file), and the specific command parameters were: trim_galore -q 20 --stringency 1 -length 35. After the adapters were removed, considering that this technology used the eclip library construction method, there was a 10bp random N barcode at the 5' end of each read, which could be used to remove duplications caused by PCR during the library construction process. We used this barcode to remove duplications from the sequencing data. Finally, FASTX-Toolkit (version 0.0.13) was used to remove the 10bp barcode in the deduplication result. The filtered data would be used for subsequent analysis.

### 2) Back-posting the data of the present application

We performed subsequent data analysis on the sequencing data. We first back-posted the sequencing data to the reference genome. The specific method was as follows. In the sequencing results of the present application, the bases A would mostly be converted into G, which was close to the ternary base data. Therefore, the first task for back-posting data was to build an environment for back-posting data based on the ternary bases. First, the A-to-G conversion in the reference genome (version number hg38) and reference transcriptome (version number GRCh38.p13; GCF _000001405.39) required for later use was carried out, and the ternary base index files of the reference genome and transcriptome required for back-posting data were constructed, respectively. Secondly, the unconverted A in the sequencing data was converted into G to obtain a complete ternary base sequencing data, and the coordinates of the unconverted A in the read segments were marked.

After the environment for back-posting data was constructed based on ternary bases, the data alignment tool STAR (version number 2.7.5.c) was used to back-paste the reads to the reference genome. The reads that were not successfully aligned would be re-extracted and then re-aligned using bowtie (version 1.3.0) to align to the transcriptome. Then, the relevant transcriptome coordinates of the reads aligned to the transcriptome were converted into genome coordinates. Subsequently, according to the coordinate information of the marked unconverted A, the G in the coordinate information corresponding to the read sequences in the two BAM format files obtained by the alignment was converted back to A. Finally, the results of the two alignments were combined to obtain a BAM format file for downstream analysis.

### 3) Analysis of A-to-G signal conversion efficiency

After obtaining the back-posted BAM file, the read coverage of all sites in the reference genome was firstly calculated based on the alignment information of the reads in the BAM file. In this process, we first converted the file from BAM format to mpileup format, and then the A-to-G signal conversion efficiency in the covered reads of all adenine A sites on each chromosome in the genome was obtained by counting according to the mpileup format, in which the median represented the A-to-G signal conversion efficiency on the chromosome. Finally, the A-to-G signal conversion efficiency on the entire genome was the median conversion efficiency of all chromosomes.

### 4) Preliminary identification of m⁶A candidate sites

After obtaining the back-posted BAM file, the preliminary identification of m⁶A candidate sites was carried out. First, based on the alignment information of the reads in the BAM file, the read coverage of all sites in the reference genome was counted. This command first converted the file from BAM format to mpileup format, and then the number information of unconverted A in the covered reads for each site in the genome was obtained by counting according to the mpileup format. Subsequently, the file obtained in the previous step was formatted into a specific fmat format file. During the format conversion process, the annotation information of the gene was also combined to mark the genomic coordinates, gene information, transcript information, information of covered reads, and number information of the unconverted A contained in the covered reads for each candidate A site on the genome. Finally, based on the above statistical information, the m⁶A candidate sites were preliminarily obtained.

### 5) False positive filtering of m⁶A candidate sites

During the development of a sodium bisulfite-based method for the detection of m5C, it was found that sodium bisulfite treatment was sensitive to the secondary structure of RNA. RNA with a complex secondary structure would cause the C on it not to be converted to T when treated with sodium bisulfite, thereby leading to false positive results. In order to avoid the same false positive results, when we initially identified m6A candidate sites, we first filtered reads containing more than three unconverted A. The number of such reads accounted for about 2% of the overall data, and the possible sources were RNAs sensitive to NaNO₂ treatment. In addition, candidate sites occurring in areas sensitive to NaNO₂ treatment would also be filtered. For an m⁶A candidate site, among all covered reads, if more than 20% of the reads contained more than three unconverted A, this candidate site was considered to occur in a NaNO₂-sensitive region, that was, the RNA transcribed in this region was not easy to occur A-to-G conversion during the NaNO₂ treatment. The above filtering could further ensure that the obtained m⁶A candidate sites were credible.

### 6) Performing statistical tests on m⁶A candidate sites

Since the A-to-G mutation rate in the present application had not reached 100%, and RNA transcribed in different regions would have different conversion efficiencies due to different sequences and secondary structures of RNA, we further filtered the candidate sites based on "signal-to-noise ratio". We performed statistical tests based on the genomic context in which m⁶A candidate sites were located, in which relative to the background, the m⁶A candidate sites whose proportion of unconverted A was significantly higher than that of the background were further selected. The specific method comprised: for each candidate m⁶A site, we used the conversion efficiency of different genes as the background to conduct a statistical test (based on binomial distribution or Poisson distribution), and calculate the significance P value of the candidate site. After selecting the candidate site with a P value less than 0.005, FDR correction was further performed. After the correction, the candidate site with a P value less than 0.005 would be used as the m⁶A candidate site for final detection in the present application.

### 1.2 Sequencing results

The following showed the cDNA library sequencing results of each RNA sample after treatment with Condition C.

### 1) The method of the present application could achieve high A-to-I conversion efficiency

In order to prove the conversion efficiency of the present application, the model sequences containing 20%, 50% and 80% m⁶A modification ratios (Spike in 2, Spike in 3 and Spike in 4) and the Spike in 1 model sequence without m⁶A modification (SEQ ID NO:9) were incorporated into the fragmented whole-cell RNA.

The preparation method of the Spike in 2 model sequence containing 20% m⁶A modification ratio comprised: the Spike in 2 model sequence containing m⁶A modification (SEQ ID NO: 10) and the Spike in 2' model sequence without m⁶A modification (SEQ ID NO:11) were mixed at a molar ratio of 2:8; the preparation method of the Spike in 3 model sequence containing 50% m⁶A modification ratio comprised: the Spike in 3 model sequence containing m⁶A modification (SEQ ID NO:12) and the Spike in 3' model sequence without m⁶A modification (SEQ ID NO: 13) were mixed at a molar ratio of 5:5; the preparation method of the Spike in 4 model sequence containing 80% m⁶A modification comprised: the Spike in 4 model sequence containing m⁶A modification (SEQ ID NO:14) and the Spike in 4' model sequence without m⁶A modification (SEQ ID NO:15) were mixed at a molar ratio of 8:2.

The nitrosation reaction and sequencing library construction were performed according to the experimental methods of Example 3. Finally, the signal conversion efficiency of A-to-G was analyzed. As mentioned before, A was converted to I after the deamination, and I underwent the amplification process to generate a G signal during sequencing. Therefore, the signal conversion of A-to-G reflected the conversion efficiency of A-to-I.

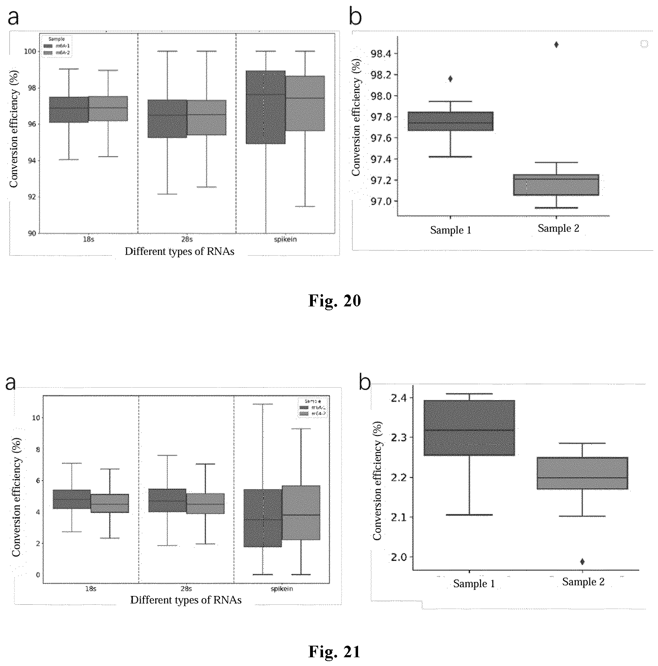

In the present application, we used the HEK293T cell line to analyze the A-to-G signal conversion efficiency of different types of RNA, such as 18S rRNA, 28S rRNA, mRNA, and incorporated model sequences. The results were shown in Fig. 20. From Fig. 20, it could be seen that on four different RNAs, the method of the present application could achieve an A-to-G signal conversion efficiency of about 97% (shown as the mutation rate in the figure), that was, the A-to-I conversion rate was high.

### 2) The method of the present application could achieve low C-to-U conversion efficiency

At the same time, the sequencing data analysis based on the present application showed that the conversion efficiency of C-to-U in different types of RNA, such as 18S rRNA, 28S rRNA, mRNA and incorporated model sequences (the sequencing results were displayed as C-to-T signals, and the analysis was also based on the statistics of the conversion ratio of C-to-T) remained at around 4%, and the results were shown in Fig. 21. These results confirmed that by using the detection method of the present application, the non-specific conversion rate of C was low and remained stable, thereby causing low interference to the m⁶A detection.

### 3) The method of the present application could specifically detect m⁶A site on RNA

In the human HEK293T cell line, we detected two known m⁶A sites on rRNA, namely m⁶A_1832 site on 18S rRNA and m⁶A_4220 site on 28S rRNA. These two sites had always been considered to have a high proportion of m⁶A modification. The detection results were shown in Fig. 22. From the high-throughput sequencing signal map of 18S rRNA displayed by IGV (the panel a in Fig. 22), it could be seen that in the m⁶A_1832 site region, the most covered reads had base A at this site, while on the surrounding non-m⁶A-modified sites, the covered reads all had base G. This showed that the method of the present application had very good detection specificity, could specifically detect the m⁶A site, and could well quantify the methylation modification level of the site. Similarly, a clear m⁶A modification signal could also be seen at the m⁶A_4220 site on 28S (the panel b in Fig. 22), and the content of A covered at this site was close to its true m⁶A modification level.

### 4) The method of the present application could specifically detect the m⁶A site on mRNA

In order to verify whether the method of the present application could detect m⁶A sites on mRNA, we detected the classic m⁶A modification sites (m⁶A site 1216 on the mRNA of gene ACTB, and m⁶A sites 967 and 1060 on the mRNA of gene INCENP) in the HEK293T cell line. Based on the known classic m⁶A sites on mRNAs, the feasibility of the method of the present application was analyzed.

The detection results were shown in Fig. 23. The results showed that obvious m⁶A signals could be detected at the known m⁶A sites in mRNAs as mentioned above.

### 5) The method of the present application could detect the distribution of m⁶A in mRNA

By analyzing the detected m⁶A sites, it was found that m⁶A was widely distributed in the 3'UTR region of mRNA. This distribution pattern in mRNA was consistent with the research results of the m⁶A distribution patterns in the existing reports, and the sequences of the detected site showed a sequence motif consistent with the known m⁶A motif DRACH (shown in Fig. 24). It showed that the method of the present application could accurately detect m⁶A in mRNA.

### 6) The method of the present application could accurately detect the modification level of m⁶A

In order to further prove that the method of the present application could accurately detect the modification level of m⁶A in the whole transcriptome, we incorporated m⁶A model sequences with different m⁶A modification ratios into the RNA samples of the present application, detected the m⁶A modification levels through the method of the present application, and compared the detection results of m⁶A model sequences with their true m⁶A modification ratios. The comparison results were shown in the panel a in Fig. 25. It could be seen from the panel that the m⁶A modification levels of the model sequences Spike in 2 and Spike in 4 observed in the present application were basically consistent with the expected levels, and were located near the diagonal line, indicating that the method of the present application could accurately detect the level of m⁶A modification in RNA. Among them, the calculated value of the m⁶A modification level of the model sequence Spike in 3 was slightly lower than the expected level, and this might be due to the fact that Spike in 3 had other similar sequences on the converted ternary base genome, which affected the back-posting process of sequencing reads, so that the later calculation showed a relatively lower modification ratio of m⁶A modification sites in Spike in 3.

The panel b in Fig. 25 showed that the present application could detect a total of more than 100,000 m⁶A sites in HEK293T cell mRNA in duplicate, and the vast majority of sites could be detected in both replicates (82%), indicating that the method of the present application had high sensitivity, stability and good reliability.

The panel c in Fig. 25 showed the comparison of modification levels in the common m⁶A modification sites detected by the method of the present application in HEK293T cell mRNA in duplicate. As shown in the panel c in Fig. 25, in the two technical replicates, the m⁶A modification levels corresponding to the m⁶A sites were basically around the diagonal line, with high consistency and a correlation coefficient of 0.96, which further illustrated that the method of the present application could stably and accurately detect the m⁶A modification level.

### 7) The method of the present application could achieve accurate detection of the m⁶A modification level under different reaction conditions

In order to further prove that the method of the present application could accurately detect the m⁶A modification level in the whole transcriptome under different reaction conditions, we performed tests under different reaction conditions, in which the reaction temperature was elevated or the composition of buffer solution was changed, and the differences in their respective conversion efficiencies were compared through high-throughput sequencing.

The model sequences containing m⁶A modification respectively at ratios 5%, 20%, 50%, and 80% (Spike in 2, Spike in 4, Spike in 5, Spike in 6), as well as Spike in 1 model sequence without m⁶A modification (SEQ ID NO:9) and Spike in 7 model sequence containing m⁶A modification (SEQ ID NO:20) were simultaneously incorporated into the fragmented whole-cell RNA.

The preparation method of the Spike in 2 model sequence containing a ratio of 5% m⁶A modification comprised: Spike in 2 model sequence containing m⁶A modification (SEQ ID NO:10) and Spike in 2' model sequence without m⁶A modification (SEQ ID NO :11) were mixed at a molar ratio of 0.5:9.5; the preparation method of the Spike in 4 model sequence containing a ratio of 20% m⁶A modification comprised: Spike in 4 model sequence containing m⁶A modification (SEQ ID NO: 14) and Spike in 4' model sequence without m⁶A modification (SEQ ID NO: 15) were mixed at a molar ratio of 2:8; the preparation method of the Spike in 5 model sequence containing a ratio of 50% m⁶A modification comprised: Spike in 5 model sequence containing m⁶A modification (SEQ ID NO:16) and Spike in 5' model sequence without m⁶A modification (SEQ ID NO:17) were mixed at a molar ratio of 5:5; the preparation method of the Spike in 6 model sequence containing a ratio of 80% m⁶A modification comprised: Spike in 6 model sequence containing m⁶A modification (SEQ ID NO: 18) and Spike in 6' model sequence without m⁶A modification (SEQ ID NO: 19) were mixed at a molar ratio of 8:2.

The nitrosation reaction and sequencing library construction were carried out according to the experimental methods of Example 3 above.

The post-processing results were shown in the panel a in Fig. 26. It could be seen from the figure that the method of the present application under all different conditions could achieve an A-to-G signal conversion efficiency of about 98%. At the same time, the panel b in Fig. 26 showed that the m⁶A modification levels of the model sequence detected by the method of the present application under different conditions were basically consistent with the expected levels, indicating that the method of the present application could accurately detect the m⁶A modification level in RNA under different conditions.

### 2. Triple tandem quadrupole mass spectrometry QQQ detection results

### 2.1 Detection results of conditions A, B, C and 1-1

The reaction conditions corresponding to conditions A, B, C, D, E and 1-1 and the A-to-I conversion rates (I/(I+A)) were shown in Table 21. The difference between Condition 1-1 and Condition C was that the concentrations and pH values of the MES buffer solution in the nitrosation system (step 2.2) were different (as shown in Table 21), and the rest were the same. The difference between Condition D and Condition C lied in the concentration of sodium nitrite used, the reaction temperature and time, and in Condition D, 1 M NaNO₂ was used, the reaction was carried out at 50°C for 0.5 hours, and the rest were the same. The difference between Condition E and Condition C lied in the type of buffer solution used in the reaction, the reaction temperature and time, and in Condition E, p-toluenesulfonic acid aqueous solution was used as the buffer solution, the reaction was carried out at 50°C for 0.5 hours, and the rest were the same.

**Table 21**

| Reaction condition | Nitrosatio n reagent | Buffer | Glyoxal concentratio n for protection | Amount of exogenously added glyoxal | Final concentration of glyoxal for nitrosation | Boric acid incubation (50 mM) | Temperature (°C) | Time (h) | I/(I+A) |
|---|---|---|---|---|---|---|---|---|---|
| A | 750 mM NaNO₂ | 40mM MES (pH6.0) | 1.3M | / | 0.52 M | Mode 1 | 16 | 8 | 95.3% |
| B | 750 mM NaNO₂ | 40mM MES (pH6.0) | 1.3M | 0.88 M | 1.4 M | Mode 1 | 16 | 8 | 100% |
| C | 750 mM NaNO₂ | 40mM MES (pH6.0) | 1.3M | 0.88 M | 1.4 M | Mode 2 | 16 | 8 | 100% |
| D | 1000 mM NaNO₂ | 40mM MES (pH6.0) | 1.3M | 0.88 M | 1.4 M | Mode 2 | 50 | 0.5 | 100% |
| E | 750 mM NaNO₂ | TsOH (pH6.0) | 1.3M | 0.88 M | 1.4 M | Mode 2 | 50 | 0.5 | 100% |
| 1-1 | 750 mM NaNO₂ | 100mM MES (pH5.5) | 1.3M | 0.88 M | 1.4 M | Mode 2 | 16 | 8 | 100% |

| | | | | | | | | | |
|---|---|---|---|---|---|---|---|---|---|
| Note: For boric acid incubation, "Mode 1" referred to adding boric acid to the nitrosation reaction system, and "Mode 2" referred to adding boric acid to the nucleic acid protection system after the nucleic acid was protected by glyoxal and before the nitrosation reaction. | | | | | | | | | |

It could be seen from Table 21 that after the treatment under conditions B, H4, and 1-1, the A-to-I conversion rates in the RNA sample were close to complete. After the treatment under Condition A, the A-to-I conversion rate in the RNA sample reached more than 95%. In addition, it could be seen from Table 21 that in the nitrosation reaction system, increasing the final concentration of glyoxal would promote the conversion of A.

### 2.2 Effects of different types of nitrosation reagents on the A-to-I conversion rate

The NaNO₂ in the nitrosation reaction system in Step 2.2 of Condition A of the above experimental method was replaced with different concentrations of KNO₂, and the specific catalytic reaction conditions and conversion rates of each of bases were shown in Table 22. The rest steps or reaction conditions not mentioned herein were the same as Condition A. G underwent non-specific conversion during the nitrosation reaction and was converted into X (xanthine). The X/G in the table represented the conversion rate of G-to-X.

**Table 22**

| Reaction condition | KNO₂ | Buffer | Time (h) | Temperature (°C) | A-to-I conversion rate | X/G |
|---|---|---|---|---|---|---|
| 1 | 1.0 M | 40mM MES (pH 5.0) | 24 | 16 | 100% | 2.1% |
| 2 | 1.0 M | 40mM MES (pH 5.5) | 24 | 16 | 100% | 1.8% |
| 3 | 0.75 M | 100mM MES (pH 5.5) | 24 | 16 | 95.2% | 1.4% |

It could be seen from Table 22 that NaNO₂, like KNO₂, could effectively catalyze the conversion of A-to-I, and the conversion rate of G remained at a low level (e.g., 2.1% or below).

### 2.3 Effect of NaNO₂ concentration and buffer solution on the A-to-I conversion rate

By adjusting the NaNO₂ concentration, buffer solution concentration and pH value in the nitrosation reaction system in Step 2.2 of the above experimental method, the effects of NaNO₂ concentration and buffer solution on the A-to-I conversion rate were studied. The specific catalytic reaction conditions and conversion rates of each base were shown in Table 23. The rest steps or reaction conditions not mentioned herein were the same as Condition B.

**Table 23**

| Reaction condition | NaNO₂ | Buffer | Time (h) | Temperature (°C) | X/G |
|---|---|---|---|---|---|
| 1 | 0.75 M | 100mM MES (pH 5.5) | 4 | 100% | 1.61% |
| 2 | 0.75 M | 100mM MES (pH 5.5) | 6 | 100% | 2.03% |
| 3 | 0.75 M | 100mM MES (pH 5.5) | 8 | 100% | 2.17% |
| 4 | 1.0 M | 40mM MES (pH 6.0) | 4 | 100% | 2.08% |
| 5 | 1.0 M | 40mM MES (pH 6.0) | 6 | 100% | 2.48% |
| 6 | 1.0 M | 40mM MES (pH 6.0) | 8 | 100% | 2.35% |
| 7 | 0.75 M | 40mM MES (pH 6.0) | 6 | 100% | 1.92% |
| 8 | 0.75 M | 40mM MES (pH 6.0) | 8 | 100% | 1.95% |

It could be seen from Table 23 that when the nitrosation reaction was carried out under the above conditions, the conversion rates of A were close to complete, and the conversion rates of G were maintained at a low level.

### 2.4 Effect of boric acid incubation mode on the A-to-I conversion rate

In this experiment, the effect of boric acid incubation mode on the A-to-I conversion rate was studied. In short, by adjusting the boric acid incubation mode in the above experimental method, the effect of the boric acid incubation mode on the A-to-I conversion rate was studied. The specific nucleic acid protection and catalytic reaction conditions, as well as the conversion rate results were shown in Table 24. The rest steps or reaction conditions not mentioned herein were the same as Condition B. Among them, "Mode 1" of boric acid incubation referred to adding boric acid to the nitrosation reaction system; "Mode 2" referred to adding boric acid to the nucleic acid protection system after the nucleic acid was protected by glyoxal and before the nitrosation reaction; "Mode 3" referred to that after the incubation of glyoxal and boric acid, the nucleic acid was protected with the incubation product of glyoxal and boric acid.

**Table 24**

| Reaction condition | MES buffer solution | H₃BO₃ | Boric acid incubation mode | Time (h) | A-to-I conversion rate | G-to-X conversion rate |
|---|---|---|---|---|---|---|
| 1 | 40 mM pH 6.0 | 50 mM | Mode 1 | 8 | 100% | 4.11% |
| 2 | 40 mM pH 6.0 | 50 mM | Mode 2 | 8 | 100% | 4.21% |
| 3 | 40 mM pH 6.0 | 50 mM | Mode 3 | 8 | 92.63% | 1.52% |

It could be seen from Table 24 that the above three boric acid incubation modes all could effectively promote the conversion of A-to-I, and the non-specific conversion rates of G were all below 5%.

### 2.5 Effect of glyoxal on the A-to-I conversion rate

In this experiment, the effects of glyoxal concentration in nucleic acid protection and nitrosation reactions on the A-to-I conversion rate were studied. Briefly, by adjusting the glyoxal concentration in the nucleic acid protection reaction system in Step 2.1 of the above experimental method and the amount of exogenously added glyoxal in the nitrosation reaction system in Step 2.2, the effects of glyoxal on the A-to-I conversion rate were studied. The specific catalytic reaction conditions and conversion rate results were shown in Table 25. The rest steps or reaction conditions not mentioned herein were the same as Condition A.

**Table 25**

| Reaction condition | Nucleic acid protection | Amount of exogenously added glyoxal | Final concentration of glyoxal in the nitrosation reaction | A-to-I conversion rate |
|---|---|---|---|---|
| 1 | / | 0.88 M | 880 mM | 77.8% |
| 2 | 1.3 M glyoxal | / | 520 mM | 98.2% |
| 3 | 1.3 M glyoxal | 0.88 M | 1.4 M | 100% |
| 4 | / | / | 0 M | 0% |

It could be seen from Table 25 that the higher the glyoxal concentration in the nitrosation reaction, the higher the conversion rate of A; in addition, compared to the sample without nucleic acid protection, the conversion rate of A in the sample protected by glyoxal was significantly higher (by comparing samples 1 and 2), it might be because that during the nucleic acid protection process, the combination of glyoxal and guanine promoted the denaturation of the nucleic acid, thereby making the conversion of A more complete.

### 2.6 Effect of nitrosation reaction time on the A-to-I conversion rate

In this experiment, the effect of nitrosation reaction time on the A-to-I conversion rate was studied. Briefly, by adjusting the reaction time in Step 2.2 Nitrosation reaction of the above experimental method, the effect of nitrosation reaction time on the A-to-I conversion rate was studied. The specific catalytic reaction conditions and conversion rate results were shown in Table 26. The rest steps or reaction conditions not mentioned herein were the same as Condition B.

**Table 26**

| Reaction condition | Glyoxal concentration (nucleic acid protection) | Amount of exogenously added glyoxal | Time (h) | Temperature (°C) | A-to-I conversion rate | G-to-X conversion rate |
|---|---|---|---|---|---|---|
| 1 | 1.3 M | 0.88 M | 4 | 16 | 100% | 2.08% |
| 2 | 1.3 M | 0.88 M | 6 | 16 | 100% | 2.48% |
| 3 | 1.3 M | 0.88 M | 8 | 16 | 100% | 1.4% |
| 4 | 1.3 M | 0.88 M | 12 | 16 | 100% | 1.6% |
| 5 | 1.3 M | 0.88 M | 16 | 16 | 100% | 1.8% |
| 6 | 1.3 M | / | 8 | 16 | 88.4% | 1.2% |
| 7 | 1.3 M | / | 12 | 16 | 95.2% | 1.6% |
| 8 | 1.3 M | / | 16 | 16 | 98.3% | 1.6% |

It could be seen from Table 26 that the higher the concentration of glyoxal in the nitrosation reaction system, the shorter the reaction time required for the nitrosation reaction. For example, when the reaction was carried out at 16°C for 4 hours using 1.3 M glyoxal for nucleic acid protection and under the conditions that 0.88 M glyoxal was exogenously added during the nitrosation reaction, the conversion rate of A was close to complete.

### 2.7 Effect of buffer solution on the A-to-I conversion rate

In this experiment, the effect of buffer solution in the nitrosation reaction system on the A-to-I conversion rate was studied. Briefly, by using different acids (e.g., H₂SO₄, H₃PO₃, TsOH) to adjust the reaction solution (without MES) in Step 2.2 Nitrosation reaction of the above experimental method to the corresponding pH values, the effects of buffer solution on the A-to-I conversion rate were studied. The specific catalytic reaction conditions and conversion rate results were shown in Table 27. The rest unmentioned steps or reaction conditions were the same as Condition A.

**Table 27**

| Reaction condition | NaNO₂ | Buffer | Time (h) | Temperature (°C) | A-to-1 conversion rate |
|---|---|---|---|---|---|
| 1 | 1.0 M | H₂SO₄(pH 6.0) | 24 | 16 | 94.8% |
| 2 | 1.0 M | H₃PO₃(pH 5.0) | 24 | 16 | 98.4% |
| 3 | 1.25 M | H₃PO₃(pH 5.0) | 24 | 16 | 98.1% |
| 4 | 1.0 M | H₃PO₃(pH 6.0) | 24 | 16 | 96.3% |
| 5 | 1.0 M | TsOH(pH 5.0) | 24 | 16 | 96.6% |
| 6 | 0.75 M | MES(pH 6.0) | 24 | 16 | 99.0% |

It could be seen from Table 27 that the above acidic solutions could all promote the conversion of A-to-I (e.g., the conversion rates were all above 94%).

### 2.8 Effect of MES concentration and pH value on the A-to-I conversion rate

In this experiment, the effects of MES concentration and pH value in the nitrosation reaction system on the A-to-I conversion rate were studied. Briefly, by adjusting the concentration and pH value of the MES buffer solution in Step 2.2 Nitrosation reaction of the above experimental method, the effects of MES concentration and pH value on the A-to-I conversion rate were studied. The specific catalytic reaction conditions and conversion rate results of each sample group were shown in Table 28. The rest steps or reaction conditions not mentioned herein were the same as Condition A.

**Table 28**

| Reaction condition | MES | Time (h) | Temperature (°C) | A-to-1 conversion rate |
|---|---|---|---|---|
| 1 | 20 mM pH5.5 | 18 | 22 | 95.63% |
| 2 | 20 mM pH6.0 | 18 | 22 | 92.4% |
| 3 | 40 mM pH5.5 | 18 | 22 | 92.2% |
| 4 | 40 mM pH6.0 | 18 | 22 | 93.5% |
| 5 | 60 mM pH6.0 | 24 | 16 | 93.1% |
| 6 | 100 mM pH5.0 | 12 | 22 | 97.5% |

It could be seen from Table 28 that in the MES buffer solution system with the above concentration and pH value, the conversion rates of A were all more than 92%.

### 2.9 Effect of final concentration of glyoxal in the nitrosation reaction on the A-to-I conversion rate

In this experiment, the effect of the final concentration of glyoxal in the nitrosation reaction on the A-to-I conversion rate was studied. In short, by adjusting the glyoxal concentration in the nucleic acid protection reaction system in Step 2.1 and the amount of exogenously added glyoxal in Step 2.2 Nitrosation reaction of the above experimental method, thereby changing the final concentration of glyoxal in the nitrosation reaction, the effect of the final concentration of glyoxal on the A-to-I conversion rate was studied. The specific catalytic reaction conditions and conversion rate results were shown in Table 29. The rest steps or reaction conditions not mentioned herein were the same as Condition B.

**Table 29**

| Reaction condition | Glyoxal concentration (nucleic acid protection) | Amount of exogenously added glyoxal | Final concentration of glyoxal in the nitrosation reaction | A-to-I conversion rate | G-to-X conversion rate |
|---|---|---|---|---|---|
| 1 | 1.3 M | 0.88 M | 1.4 M | 99.67% | 1.90% |
| 2 | 330 mM | 0.88 M | 1.0 M | 96.56% | 1.80% |
| 3 | 80 mM | 0.88 M | 0.91 M | 89.71% | 1.56% |

It could be seen from Table 29 that the higher the glyoxal concentration in the nitrosation reaction system, the higher the conversion rate of A accordingly.

### 2.10 Effect of nitrosation reaction temperature on the A-to-I conversion rate

In this experiment, the effect of nitrosation reaction temperature on the A-to-I conversion rate was studied. In short, by adjusting the reaction temperature and time in Step 2.2 Nitrosation reaction of the above experimental method, the effect of nitrosation reaction temperature on the A-to-I conversion rate was studied. The specific nucleic acid protection and catalytic reaction conditions and conversion rate results were shown in Table 30. The rest steps or reaction conditions not mentioned herein were the same as Condition C.

**Table 30**

| Reaction condition | Glyoxal concentration (nucleic acid protection) | Amount of exogenously added glyoxal | Time (h) | Temperature (°C) | A-to-I conversion rate | G-to-X conversion rate |
|---|---|---|---|---|---|---|
| 1 | 1.3 M | 0.22 M | 2 | 16 | 71.7% | 0.90% |
| 2 | 1.3 M | 0.44 M | 2 | 16 | 82.2% | 1.10% |
| 3 | 1.3 M | 0.88 M | 8 | 16 | 100% | 3.3% |
| 4 | 1.3 M | 0.44 M | 2 | 50 | 100% | 3.3% |
| 5 | 1.3 M | 0.88 M | 0.5 | 50 | 100% | 2.5% |

It can be seen from Table 30 that the nitrosation reaction could occur in the temperature range of 16 to 50°C. The higher the temperature, the shorter the reaction time required. Furthermore, lowering the reaction temperature and shortening the reaction time were beneficial to reducing the G-to-X conversion rate.

### 2.11 Effect of carbonyl compound type on the A-to-I conversion rate

In this experiment, the effect of the type of carbonyl compound on the A-to-I conversion rate was studied. In short, the glyoxal used in Step 2.2 Nitrosation reaction of the above experimental method was replaced with a carbonyl compound of different types and concentrations, and the effect of the type of carbonyl compound on the A-to-I conversion rate was studied. The specific nucleic acid protection and catalytic reaction conditions and conversion rate results were shown in Table 31. The rest steps or reaction conditions not mentioned herein were the same as Condition C.

**Table 31**

| Reaction condition | Nucleic acid protection (glyoxal) | Exogenously added carbonyl compound for nitrosation reaction | Tim e (h) | Temperature (°C) | A-to-I conversio n rate | G-to-X conversion rate |
|---|---|---|---|---|---|---|
| 1 | 1.3 M | 200, mM 2-bromomalondialdehyde | 8 | 16 | 100% | 5.64% |
| 2 | 1.3 M | 200 mM 2-bromomalondialdehyde | 8 | 16 | 100% | 4.96% |
| 3 | 1.3 M | 400, mM 2-bromomalondialdehyde | 8 | 16 | 84.47% | 4.33% |
| 4 | 1.3 M | 400, mM 2-bromomalondialdeyehyde | 4 | 16 | 100% | 5.00% |
| 5 | 1.3 M | 400 mM trichloroacetaldehyde | 8 | 16 | 100% | 5.14% |
| 6 | 1.3 M | 0.88 M glyoxal | 8 | 16 | 100% | 4.40% |

It could be seen from Table 31 that after using high-concentration glyoxal (e.g., 1.3 M glyoxal) for nucleic acid protection, adding different carbonyl compounds (e.g., 2-bromomalondialdehyde, trichloroacetaldehyde, etc.) to the nitrosation reaction system could always promote the conversion of A-to-I. In addition, in Reaction Condition 6, the imine intermediate produced during the glyoxal-catalyzed nitrosation reaction was detected, and its mass spectrum was shown in Fig. 27.

### 2.12 Effect of boric acid or salt thereof on the A-to-I conversion rate

In this experiment, the effect of boric acid or salt thereof in the nitrosation reaction on the A-to-I conversion rate was studied. Briefly, the boric acid in Step 2.2 Nitrosation reaction of the above experimental method was replaced with potassium borate of different pH values, and the effect of boric acid or salt thereof on the A-to-I conversion rate was studied. The specific catalytic reaction conditions and conversion rate results were shown in Table 32. The rest steps or reaction conditions not mentioned herein were the same as Condition C.

**Table 32**

| Reaction condition | 50 mM K₃BO₃ | Buffer | Time (h) | Temperature (°C) | A-to-I conversion rate | G-to-X conversion rate |
|---|---|---|---|---|---|---|
| 1 | pH6.0 | 100 mM MES(pH 5.5) | 4 | 16 | 100% | 1.59% |
| 2 | pH6.0 | 100 mM MES(pH 5.5) | 8 | 16 | 100% | 1.91% |
| 3 | pH7.0 | 100 mM MES(pH 5.5) | 4 | 16 | 100% | 1.35% |
| 4 | pH7.0 | 100 mM MES(pH 5.5) | 8 | 16 | 100% | 1.70% |
| 5 | pH8.9 | 40 mM MES(pH 6.0) | 8 | 16 | 100% | 1.56% |

It could be seen from Table 32 that after the boric acid in the nitrosation reaction was replaced with borate, the A-to-I conversion rate was not affected (both were close to complete).

Although the specific embodiments of the present application have been described in detail, those skilled in the art will understand that various modifications and changes can be made to the details based on all the teachings that have been published, and these changes are within the protection scope of the present application. The entirety of the present application is given by the appended claims and any equivalents thereof.

## Claims

1. A method for detecting N6-methyladenine in a nucleic acid molecule, which comprises the following steps:
(1) performing protection of an amino group of guanine in a nucleic acid molecule to be detected to obtain a protected nucleic acid molecule;
(2) reacting the protected nucleic acid molecule with a nitrite in the presence of a carbonyl compound to convert an adenine in the nucleic acid molecule to be detected into a hypoxanthine;
(3) optionally, performing deprotection of the product obtained in step (2);
(4) detecting the product obtained in the previous step.

2. The method according to claim 1, wherein in step (1), a second carbonyl compound is used to protect the amino group of guanine in the nucleic acid molecule to be detected;
preferably, the carbonyl compound is from:
(i) a second carbonyl compound added in step (1);
(ii) a first carbonyl compound added in step (2); or,
(iii) a combination of (i) and (ii);
preferably, the second carbonyl compound is the same as or different from the first carbonyl compound; more preferably, the second carbonyl compound is the same as the first carbonyl compound;
preferably, the step (2) does not comprise the step of adding the first carbonyl compound, and the carbonyl compound is from the second carbonyl compound added in step (1); or, step (2) comprises a step of adding the first carbonyl compound, and the first carbonyl compound is the same as or different from the second carbonyl compound;
preferably, in step (3), the guanine in the product is optionally undergoes deprotection;
preferably, the first carbonyl compound and the second carbonyl compound are each independently selected from the group consisting of the compounds represented by Formula I and any combination thereof, 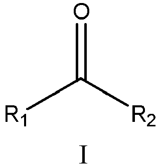
wherein R₁ is an aldehyde group, C₁₋₃ alkyl (e.g., methyl, ethyl or n-propyl), or halogen-substituted C₁₋₃ alkyl (e.g., trichloromethyl, trifluoromethyl), R₂ is H, C₁₋₃ alkyl (e.g., methyl, ethyl or n-propyl), phenyl or HC(=O)-CH(Br)-; or,
R₁ and R₂ together with the carbonyl group to which they are bonded form a cyclic structure, such as ninhydrin;
preferably, the first carbonyl compound and the second carbonyl compound are each independently selected from the group consisting of: glyoxal, 2,3-butanedione, ninhydrin, 2-bromomalonaldehyde, pyruvaldehyde, trichloroacetaldehyde, phenylglyoxal and any combination thereof;
preferably, the first carbonyl compound is selected from the group consisting of: glyoxal, 2,3-butanedione, ninhydrin, 2-bromomalondialdehyde, pyruvaldehyde, trichloroacetaldehyde, phenylglyoxal and any combination thereof;
preferably, the first carbonyl compound is selected from the group consisting of glyoxal, 2-bromomalondialdehyde, 2,3-butanedione, phenylglyoxal, trichloroacetaldehyde, ninhydrin and any combination thereof;
preferably, the second carbonyl compound is selected from the group consisting of: glyoxal, ninhydrin and any combination thereof.

3. The method according to claim 2, wherein in step (1), the nucleic acid molecule to be detected is contacted with the second carbonyl compound in a first solvent;
preferably, the first solvent is water;
preferably, in step (1), the nucleic acid molecule to be detected is contacted with the second carbonyl compound in the first solvent, and in the presence of dimethylsulfoxide (DMSO) or N,N-dimethylformamide (DMF);
preferably, in step (1), the nucleic acid molecule to be detected is contacted with the second carbonyl compound in the first solvent, and in the presence of dimethylsulfoxide (DMSO) and boric acid or salt thereof (e.g., potassium borate);
preferably, in step (1), the nucleic acid molecule to be detected is contacted with the second carbonyl compound in the first solvent, and in the presence of N,N-dimethylformamide (DMF) and boric acid or salt thereof (e.g., potassium borate);
preferably, the dimethylsulfoxide (DMSO) has a final concentration of 20 v/v% to 90 v/v%, such as about 30 v/v%, about 40 v/v%, or about 50 v/v%., about 60 v/v%, about 70 v/v%, about 80 v/v%;
preferably, the boric acid or salt thereof (e.g., potassium borate) has a final concentration of the SmM to 150 mM (e.g., SmM to 10 mM, 10 mM to 50 mM, 50 mM to 80 mM, 80 mM to 100 mM);
preferably, the second carbonyl compound has a final concentration of greater than or equal to 20 mM, preferably 20 mM to 3M (e.g., 20 mM to 100 mM, 100 mM to 500 mM, 500 mM to 1000 mM, 1M to 1.5M, 1.5 to 2M, 2M to 2.6M, such as about 0.8M, about 1.3M, about 1.8M);
preferably, in step (1), the nucleic acid molecule to be detected is contacted with the second carbonyl compound at a temperature of 16 to 60°C (e.g., about 20°C, about 25°C, about 30°C, about 35°C, about 40°C, about 45°C, about 50°C, about 55°C);
preferably, in step (1), the nucleic acid molecule to be detected is contacted with the second carbonyl compound at a temperature of 16 to 60°C (e.g., about 20°C, about 25°C, about 30°C, about 35°C, about 40°C, about 45°C, about 50°C, about 55°C) for 15 to 60 minutes (e.g., about 20 minutes, about 30 minutes, about 40 minutes, about 50 minutes).

4. The method according to any one of claims 1 to 3, wherein the method further comprises: before step (1), a step of performing pretreatment of the nucleic acid molecule to be detected;
preferably, the pretreatment comprises purification, fragmentation, denaturation, or any combination thereof of the nucleic acid molecule to be detected;
preferably, the pretreatment comprises denaturation of the nucleic acid molecules to be detected using a formamide aqueous solution or an alkali solution.

5. The method according to any one of claims 1 to 4, wherein in step (2), in the presence of the carbonyl compound, the protected nucleic acid molecule is reacted with the nitrite in a second solvent;
preferably, the second solvent is selected from the group consisting of water, p-toluenesulfonic acid aqueous solution, phosphoric acid aqueous solution, 2-(N-morpholino)ethanesulfonic acid (MES) buffer solution, sodium acetate buffer solution, 3-(N-morpholino)propanesulfonic acid (MOPS) buffer solution, piperazine-1,4-diethanesulfonic acid (PIPES) buffer solution, 4-hydroxyethylpiperazineethanesulfonic acid (HEPPS) buffer solution, or tris(hydroxymethyl)aminomethane (TRIS)) buffer solution, and any combination thereof;
preferably, the second solvent is p-toluenesulfonic acid aqueous solution, phosphoric acid aqueous solution, 2-(N-morpholino)ethanesulfonic acid (MES) buffer solution, or sodium acetate buffer solution;
preferably, the second solvent is a p-toluenesulfonic acid aqueous solution at a pH of 4.5 to 6.5 (e.g., 5 to 6);
preferably, the second solvent is a phosphoric acid aqueous solution at a pH of 4.5 to 6.5 (e.g., 5 to 6);
preferably, the second solvent is a 2-(N-morpholino)ethanesulfonic acid (MES) buffer solution, wherein the 2-(N-morpholino)ethanesulfonic acid (MES) has a final concentration of 20 mM to 750 mM (e.g., about 40 mM, about 80 mM, about 100 mM, about 150 mM, about 200 mM, about 250 mM, about 500 mM);
preferably, the second solvent is a sodium acetate buffer solution, wherein the sodium acetate has a final concentration of 300 mM to 400 mM;
preferably, in step (2), in the presence of the carbonyl compound, the protected nucleic acid molecule is reacted with the nitrite in the second solvent, and in the presence of boric acid or a salt thereof (e.g., potassium borate);
preferably, the carbonyl compound in the second solvent has a total molar concentration (final concentration) of greater than or equal to 50 mM, such as greater than or equal to 250 mM (e.g., 50 to 2000 mM, 50 to 200 mM, 200 to 500 mM, 500 to 800 mM, 800 to 1200 mM, 1200 to 1400 mM, 1400 to 1800 mM, 1800 to 2000 mM, such as 105mM);
preferably, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, such as 105 mM), and pyruvaldehyde at a final concentration of 50 to 1500 mM (e.g., 50 to 60 mM, 60 to 100 mM, 100 to 300 mM, 300 to 600 mM, 600 to 800 mM, 800 to 1200 mM, 1200 to 1500 mM);
preferably, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, such as 105 mM), and 2,3-butanedione at a final concentration of 50 to 2500 mM (e.g., 50 to 100 mM, 100 to 200 mM, 200 to 500 mM, 500 to 800 mM, 800 to 1200 mM, 1200 to 1500 mM, 1500 to 1800 mM, 1800 to 2200 mM, 2200 to 2500 mM);
preferably, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, such as 105 mM), and ninhydrin at a final concentration of 20 to 1500 mM (e.g., 20 to 100 mM, 100 to 200 mM, 200 to 500 mM, 500 to 800 mM, 800 to 1200 mM, 1200 to 1500 mM);
preferably, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, such as 105 mM), and 2-bromomalondialdehyde at a final concentration of 50 to 1500 mM (e.g., 50 to 100 mM, 100 to 200 mM, 200 to 400 mM, 400 to 500 mM, 500 to 800 mM, 800 to 1200 mM, 1200 to 1500 mM);
preferably, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, such as 105 mM) and trichloroacetaldehyde at a final concentration of 50 to 1500 mM (e.g., 50 to 100 mM, 100 to 400 mM, 400 to 500 mM, 500 to 800 mM, 800 to 1200 mM, 1200 to 1500 mM);
preferably, the second solvent contains glyoxal at a final concentration of 50 to 2000 mM (e.g., 50 to 200 mM, 200 to 500 mM, 500 to 800 mM, 800 to 1200 mM, 1200 to 1400 mM, 1400 to 1800 mM, 1800 to 2000 mM, such as 105 mM) and phenylglyoxal at a final concentration of 50 to 1500 mM (e.g., 50 to 100 mM, 100 to 400 mM, 400 to 500 mM, 500 to 800 mM, 800 to 1200 mM, 1200 to 1500 mM);
preferably, the second solvent contains glyoxal at a final concentration greater than or equal to 50 mM, such as greater than or equal to 250 mM (e.g., 50 to 200 mM, 200 to 500 mM, 500 to 800 mM, 800 to 1200 mM, 1200 to 1400 mM, 1400 to 1800 mM, 1800 to 2000 mM, such as 105mM);
preferably, the boric acid or salt thereof (e.g., potassium borate) has a final concentration of 5 mM to 150 mM (e.g., 5 mM to 10 mM, 10 mM to 50 mM, 50 mM to 80 mM, 80 mM to 100 mM);
preferably, the nitrite is selected from the group consisting of sodium nitrite, potassium nitrite and a combination of the two;
preferably, the nitrite has a final concentration of 0.5 M to 1.5 M, such as 0.5 M to 0.8 M, 0.8 to 1.25 M.

6. The method according to claim 5, wherein in step (2), in the presence of the carbonyl compound, the protected nucleic acid molecule is reacted with the nitrite in the second solvent, and in the presence of boric acid or salt thereof (e.g., potassium borate);
preferably, the boric acid or salt thereof (e.g., potassium borate) is from:
(i) a boric acid or salt thereof (e.g., potassium borate) added in step (1);
(ii) a boric acid or salt thereof (e.g., potassium borate) added in step (2);
(iii) a boric acid or salt thereof (e.g., potassium borate) added after step (1) but before step (2); or,
(iv) any combination of (i) to (iii);
preferably, step (2) does not comprise a step of adding boric acid or salt thereof (e.g., potassium borate); or, step (2) comprises a step of adding boric acid or salt thereof (e.g., potassium borate), and the boric acid or salt thereof (e.g., potassium borate) added in step (2) is the same as or different from the boric acid or salt thereof (e.g., potassium borate) added in step (1).

7. The method according to any one of claims 1 to 6, in step (2), the protected nucleic acid molecule is reacted with the nitrite at a temperature of 12 to 60°C (e.g., 16 to 60°C, 12 to 24°C, 24 to 40°C, 40 to 60°C, about 16°C, about 20°C, about 25°C, about 30°C, about 35°C, about 40°C, about 45°C, about 50°C, about 55°C);
preferably, in step (2), the protected nucleic acid molecule is reacted with the nitrite at a temperature of 12 to 60°C (e.g., 16 to 60°C, 12 to 24°C, 24 to 40°C, 40 to 60°C, about 16°C, about 20°C, about 25°C, about 30°C, about 35°C, about 40°C, about 45°C, about 50°C, about 55°C) for 10 minutes to 24 hours (e.g., 30 minutes to 24 hours, 10 minutes to 20 minutes, 20 minutes to 1 hour, 1 hour to 5 hours, 5 hours to 10 hours, 10 hours to 24 hours, about 20 minutes, about 30 minutes, about 40 minutes, about 50 minutes, about 8 hours);
preferably, in step (2), the protected nucleic acid molecule is reacted with the nitrite at a temperature of 12 to 24°C (e.g., about 16°C) for 5 hours to 10 hours (e.g., about 8 hours);
preferably, in step (2), the protected nucleic acid molecule is reacted with the nitrite at a temperature of 40 to 60°C (e.g., about 50°C) for 20 minutes to 1 hour (e.g., about 30 minutes).

8. The method according to any one of claims 1 to 7, the method further comprising: before step (4), a step of performing purification, reverse transcription, and/or amplification of the product obtained in the previous step;
preferably, the method further comprises: an additional step of detecting the nucleic acid molecule to be detected;
preferably, in step (4), the detection comprises analyzing nucleotide composition by sequencing or hybridization, mass spectrometry (e.g., triple tandem quadrupole mass spectrometry), enzymatic fragmentation, and/or chromatography;
preferably, the detection comprises analyzing nucleotide composition by sequencing;
preferably, the method further comprises comparing the detection results of step (4) with the detection results of the additional step (e.g., performing comparison of the sequence or nucleotide composition of the product in step (4) and the sequence or nucleotide composition of the nucleic acid molecule to be detected obtained in the additional step, and determining the content and/or position information of No-methyladenine in the nucleic acid molecule to be detected).

9. The method according to any one of claims 1 to 8, wherein the nucleic acid molecule to be detected is an RNA, a DNA or a DNA/RNA hybrid;
preferably, in step (3), the product obtained in step (2) undergoes deprotection under an alkaline condition; or, the product obtained in step (2) undergoes deprotection in a phosphate buffer solution;
preferably, in step (3), the product obtained in step (2) undergoes deprotection by a heat treatment under an alkaline condition;
preferably, the product obtained in step (2) undergoes deprotection by a heat treatment in a triethylamine acetate (TEAA) buffer solution containing formamide at a pH of 8 to 9 or in a phosphate buffer solution containing dimethylsulfoxide (DMSO) at a pH of 7.1 to 8;
preferably, the product obtained in step (2) undergoes deprotection by a heat treatment in a triethylamine acetate (TEAA) buffer solution containing 45 to 50 v/v% formamide at a pH of 8 to 9 or in a phosphate buffer solution containing 40 to 60 v/v % dimethylsulfoxide (DMSO) at a pH of 7.1 to 8;
preferably, the heat treatment comprises:
a) treating the product obtained in step (2) at a temperature of 80 to 95°C for 5 to 10 minutes, or,
b) treating the product obtained in step (2) at a temperature of 60 to 70°C for 1.5 to 2.5 hours (e.g., about 2 hours);
preferably, the product obtained in step (2) undergoes deprotection by the heat treatment as described in a) in a triethylamine acetate (TEAA) buffer solution containing about 47.5 v/v% formamide at a pH of about 8.6, or,
the product obtained in step (2) undergoes deprotection by the heat treatment as described in b) in a phosphate buffer solution containing about 50 v/v % dimethylsulfoxide (DMSO) at a pH of about 7.4.

10. The method according to claim 9, wherein the nucleic acid molecule to be detected is an RNA or a DNA/RNA hybrid;
preferably, in step (3), the product obtained in step (2) undergoes deprotection under an alkaline condition;
preferably, in step (3), the product obtained in step (2) undergoes a first deprotection under an alkaline condition, and the product obtained by the first deprotection undergoes a second deprotection in a phosphate buffer solution;
preferably, in step (3), the product obtained in step (2) undergoes deprotection by a heat treatment under an alkaline condition;
preferably, in step (3), the product obtained in step (2) undergoes a first deprotection by a heat treatment in a triethylamine acetate (TEAA) buffer solution containing formamide at a pH of 8 to 9;
preferably, in step (3), the product obtained in step (2) undergoes a first deprotection by a heat treatment in a triethylamine acetate (TEAA) buffer solution containing formamide at a pH of 8 to 9, and the product obtained by the first deprotection undergoes a second deprotection by a heat treatment in a phosphate buffer solution containing dimethylsulfoxide (DMSO) at a pH of 7.1 to 8;
preferably, in step (3), the product obtained in step (2) undergoes a first deprotection by a heat treatment in a triethylamine acetate (TEAA) buffer solution containing 45 to 50 v/v% formamide at a pH of 8 to 9;
preferably, in step (3), the product obtained in step (2) undergoes a first deprotection by a heat treatment in a triethylamine acetate (TEAA) buffer solution containing 45 to 50 v/v% formamide at a pH of 8 to 9, and the product obtained by the first deprotection undergoes a second deprotection by a heat treatment in a phosphate buffer solution containing 40 to 60 v/v% dimethylsulfoxide (DMSO) at a pH of 7.1 to 8;
preferably, the heat treatment comprises:
a) treating the product obtained in step (2) at a temperature of 80 to 95°C for 5 to 10 minutes, or,
b) treating the product obtained in step (2) at a temperature of 60 to 70°C for 1.5 to 2.5 hours (e.g., about 2 hours);
preferably, in step (3), the product obtained in step (2) undergoes a first deprotection by the heat treatment as described in a) in a triethylamine acetate (TEAA) buffer solution containing about 47.5 v/v% formamide at a pH of about 8.6;
preferably, in step (3), the product obtained in step (2) undergoes a first deprotection by the heat treatment as described in a) in a triethylamine acetate (TEAA) buffer solution containing about 47.5 v/v% formamide at a pH of about 8.6, and the product obtained by the first deprotection undergoes a second deprotection by the heat treatment as described in b) in a phosphate buffer solution containing about 50 v/v % dimethylsulfoxide (DMSO) at a pH of about 7.4.

11. The method according to claim 9, wherein the nucleic acid molecule to be detected is a DNA or a DNA/RNA hybrid;
preferably, in step (3), the product obtained in step (2) undergoes deprotection under an alkaline condition;
preferably, in step (3), the product obtained in step (2) undergoes deprotection by a heat treatment under an alkaline condition;
preferably, the product obtained in step (2) undergoes deprotection by a heat treatment in a triethylamine acetate (TEAA) buffer solution containing formamide at a pH of 8 to 9;
preferably, the product obtained in step (2) undergoes deprotection by a heat treatment in a triethylamine acetate (TEAA) buffer solution containing 45-50 v/v% formamide at a pH of 8 to 9;
preferably, the heat treatment comprises: treating the product obtained in step (2) at a temperature of 80 to 95°C for 5 to 10 minutes;
preferably, the product obtained in step (2) undergoes deprotection by the heat treatment as described in a) in a triethylamine acetate (TEAA) buffer solution containing about 47.5 v/v% formamide at a pH of about 8.6.

12. A kit, which comprises a first carbonyl compound and a nitrite; preferably, the first carbonyl compound is selected from the group consisting of the compounds represented by Formula I and any combination thereof, 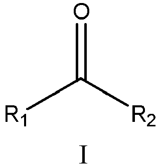
wherein, R₁ is an aldehyde group, C₁₋₃ alkyl (e.g., methyl, ethyl or n-propyl), or halogen-substituted C₁₋₃ alkyl (e.g., trichloromethyl, trifluoromethyl), R₂ is H, C₁₋₃ alkyl (e.g., methyl, ethyl or n-propyl), phenyl or HC(=O)-CH(Br)-; or,
R₁ and R₂ together with the carbonyl to which they are bonded form a cyclic structure, such as ninhydrin;
preferably, the first carbonyl compound is selected from the group consisting of: glyoxal, 2,3-butanedione, ninhydrin, 2-bromomalondialdehyde, pyruvaldehyde, trichloroacetaldehyde, phenylglyoxal and any combination thereof;
preferably, the first carbonyl compound is selected from the group consisting of: glyoxal, 2,3-butanedione, ninhydrin, 2-bromomalondialdehyde, pyruvaldehyde, trichloroacetaldehyde, phenylglyoxal and any combination thereof;
preferably, the first carbonyl compound is selected from the group consisting of glyoxal, 2-bromomalondialdehyde, 2,3-butanedione, phenylglyoxal, trichloroacetaldehyde, ninhydrin and any combination thereof;
preferably, the kit further comprises a second carbonyl compound; preferably, the second carbonyl compound is selected from the group consisting of the compounds represented by formula I and any combination thereof, 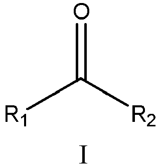
wherein R₁ is an aldehyde group, C₁₋₃ alkyl (e.g., methyl, ethyl or n-propyl), or halogen-substituted C₁₋₃ alkyl (e.g., trichloromethyl, trifluoromethyl), R₂ is H, C₁₋₃ alkyl (e.g., methyl, ethyl or n-propyl), phenyl or HC(=O)-CH(Br)-; or,
R₁ and R₂ together with the carbonyl group to which they are bonded form a cyclic structure, such as ninhydrin;
preferably, the first carbonyl compound is the same as or different from the second carbonyl compound; more preferably, the second carbonyl compound is the same as the first carbonyl compound;
preferably, the second carbonyl compound is selected from the group consisting of: glyoxal, ninhydrin and any combination thereof;
preferably, the nitrite is selected from sodium nitrite, potassium nitrite and a combination of the two;
preferably, the kit further comprises boric acid or salt thereof (e.g., potassium borate);
preferably, the kit further comprises dimethylsulfoxide (DMSO), N,N-dimethylformamide (DMF) or a combination thereof;
preferably, the kit further comprises at least one substance selected from the group consisting of: p-toluenesulfonic acid, phosphoric acid, 2-(N-morpholino)ethanesulfonic acid (MES), sodium acetate, 3-(N-morpholino)propanesulfonic acid (MOPS), piperazine-1,4-diethanesulfonic acid (PIPES), 4-hydroxyethylpiperazineethanesulfonic acid (HEPPS), and tris(hydroxymethyl)aminomethane (TRIS);
preferably, the kit further comprises at least one substance selected from the group consisting of: 2-(N-morpholino)ethanesulfonic acid (MES), sodium acetate, 3-(N-morpholino)propanesulfonic acid (MOPS), piperazine-1,4-diethyl sulfonic acid (PIPES), 4-hydroxyethylpiperazineethanesulfonic acid (HEPPS), and tris(hydroxymethyl)aminomethane (TRIS);
preferably, the kit further comprises a substance selected from the group consisting of: a substance for preparing a 2-(N-morpholino)ethanesulfonic acid (MES) buffer solution, a substance for preparing a sodium acetate buffer solution, a substance for preparing a 3-(N-morphino)propanesulfonic acid (MOPS) buffer solution, a substance for preparing a piperazine-1,4-diethanesulfonic acid (PIPES) buffer solution, a substance for preparing a 4-hydroxyethylpiperazineethanesulfonic acid (HEPPS) buffer solution, a substance for preparing a tris(hydroxymethyl)aminomethane (TRIS) buffer solution, and any combination thereof;
preferably, the kit further comprises a 2-(N-morpholino)ethanesulfonic acid (MES) buffer solution, a sodium acetate buffer solution, a 3-(N-morpholino)propanesulfonic acid (MOPS) buffer solution, a piperazine-1,4-diethanesulfonic acid (PIPES) buffer solution, a 4-hydroxyethylpiperazinoethanesulfonic acid (HEPPS) buffer solution or a tris(hydroxymethyl)aminomethane (TRIS) buffer solution or any combination thereof;
preferably, the kit further comprises a substance selected from the following: a substance for preparing a phosphate buffer solution, a substance for preparing a triethylamine acetate (TEAA) buffer solution, and combinations of the two;
preferably, the kit further comprises a phosphate buffer solution, a triethylamine acetate (TEAA) buffer solution or a combination thereof;
preferably, the triethylamine acetate (TEAA) buffer solution comprises 45 to 50 v/v% formamide, and has a pH of 8 to 9;
preferably, the phosphate buffer solution comprises 40 to 60 v/v% dimethylsulfoxide (DMSO), and has a pH of 7.1 to 8;
preferably, the kit further comprises a reagent for mass spectrometry detection and/or DNA sequencing;
preferably, the kit further comprises an instruction; preferably, the instruction describes the method according to any one of claims 1 to 11.

13. Use of the kit according to claim 12 in detecting N6-methyladenine in a nucleic acid molecule.
